# EUROPEAN PATENT SPECIFICATION

(11) **EP 2 282 645 B1**
(45) Date of publication and mention of the grant of the patent: **15.04.2015**
(21) Application number: 09753558.7
(22) Date of filing: 25.05.2009
(51) Int. Cl.: A23G 4/06, A23G 4/20, A61K 9/00

(54) **FLAVOR IMPREGNATION OF A CHEWING GUM CORE**
GESCHMACKSIMPRÄGNIERUNG EINES KAUGUMMIKERNS
IMPRÉGNATION D ARÔME D UN NOYAU DE GOMME À MÂCHER

(30) Priority: 26.05.2008 WO PCT/DK2008/000196
(43) Date of publication of application: 16.02.2011
(73) Proprietor: Fertin Pharma A/S, 7100 Vejle (DK)
(72) Inventor: THORSEN, Jesper, DK-8765 Klovborg (DK); PEDERSEN, Kurt, Moller, DK-7323 Give (DK); BANG-MADSEN, Birgitte, DK-8766 Norre Snede (DK)
(74) Representative: Patentgruppen
(86) International application number: PCT/DK2009/000116
(87) International publication number: WO 2009/143845

(56) References cited:
- EP-A- 1 474 995
- WO-A-02/102357
- WO-A-2004/004479
- WO-A-2004/004480
- WO-A-2006/124366
- WO-A-2006/127293
- WO-A-2008/027251
- WO-A-2008/055006
- JP-A- 56 137 850
- US-A1- 2002 064 576
- US-A1- 2002 164 398

## Description

### FIELD OF THE INVENTION

The invention relates to the field of chewing gum and in particular to flavor impregnation of a chewing gum core.

### TECHNICAL BACKGROUND AND PRIOR ART

Chewing gum typically comprises flavor. Flavor is used to adjust the taste of the chewing gum. In particular, nicotine-containing chewing gum may need the addition of comparatively large amounts of flavor to mask the unpleasant taste of nicotine itself. To achieve an advantageous flavor release profile from a nicotine containing chewing gum, it may be desirable to add considerable amounts of flavor to the chewing gum core, that is, the part of the chewing gum containing gum base.

The amount of flavor applicable to a chewing gum core may be limited due to process demands. Adding flavor to the gum base comprised in the chewing gum core may negatively affect the further processing of the chewing gum core. For example, the softening of the chewing gum core material by flavor may put a certain limit to the amount of flavor applicable for practical reasons. If the chewing gum core material gets too soft, it may stick to rollers in the production line and it may be difficult to punch out small pieces of individual chewing gum cores.

The coating of the chewing gum cores may also prove to be problematic, if the chewing gum cores are too soft.

US 2006/0275344 A1 discloses a method of coating a chewing gum core with a polymer film coat comprising flavor and with a further hard coating.

However, the introduction of an extra film layer surrounding the chewing gum core does not raise the flavor level in the chewing gum core to achieve prolonged flavor release. On the contrary, the flavor is confined to the film coating.

WO 2008/055006 A discloses the use of porous structures loaded with flavor to modify the flavor release from a chewing gum. The porous structures are loaded with flavor and then the loaded particles are mixed with the gum base. From this mixture, chewing gum with advantageous flavor release properties may be produced.

US 2002/064576 A1 discloses the use of a crosslinked hydroxypropylcellulose as a flavor-carrier matrix to obtain an improved flavor duration from a chewing gum. The matrix is prepared and loaded with flavor. Then the flavor carrying matrix is mixed with the gum base. From this mixture, chewing gum with advantageous flavor release properties may be produced.

In both the above mentioned cases, the flavor is confined to special structures then mixed into the gum base, adding extra process steps and material costs to the chewing gum so produced.

It is one of several objects of the present invention to provide a method of adding flavor to a chewing gum core in a costeffective way without compromising the processabillity of the chewing gum core material.

### SUMMARY OF THE INVENTION

The invention relates in a first aspect to a method of adding flavor to a chewing gum core of a chewing gum according to claim 1,
said chewing gum comprising active pharmaceutical ingredients,
said method of adding flavor to a chewing gum core comprising the steps of:
- providing a chewing gum core comprising gum base
- impregnating said chewing gum core by adding at least one dose of liquid flavor mixture onto the chewing gum core prior to any optional coating of the chewing gum core,
wherein at least the flavor in the liquid flavor mixture has affinity for the gum base in the chewing gum core,
wherein said chewing gum core comprises at least a part of said active pharmaceutical ingredient.

The term active pharmaceutical ingredient (API) covers pharmaceutically active ingredients in general, examples of which are found below.

The term *affinity for* herein covers the driving force making a liquid material suitable for an impregnation process defined below. A component of a liquid flavor mixture having affinity for a chewing gum core thus means that there is a force prompting the liquid flavor mixture component to start migrating into the chewing gum core. The force may be of physical and/or chemical nature.

The term *liquid flavor mixture* covers a liquid comprising at least one flavor having affinity for the chewing gum core. The liquid flavor mixture may comprise multiple flavors and optionally other compounds such as e.g. softeners and solvents. Examples of liquid flavor mixtures according to provisions of the invention are given below, e.g. in the examples.

The term *impregnation* herein covers a process by which a liquid material is applied to a solid material characterized in that the liquid material after contacting the solid will partly or fully penetrate the solid with time. In the present context, impregnation does not mean simple absorption of a liquid into the pores of a porous solid but rather the migration of components of the liquid material into the solid material. For example, a hydrophobic liquid flavor mixture component will have a tendency to migrate into the hydrophobic parts of a chewing gum core during and/or after the impregnation step(s) is/has been carried out. This tendency of hydrophobic flavor molecules to migrate into the hydrophobic parts of the chewing gum core means that the hydrophobic flavor molecules have affinity for the hydrophobic parts of the chewing gum core, the term *affinity for* being defined as above.

The term *chewing gum core* herein covers the part of the chewing gum containing gum base. The impregnation method described herein is applied on the chewing gum core, that is, after the chewing gum core components have been mixed by conventional methods and the chewing gum core has been formed to have a suitable shape and a desired dimension. The impregnation of the chewing gum core with liquid flavor mixture is applied prior to any optional coating process known in the art.

When the chewing gum is obtained by compression techniques, the individual granules comprising gum base may be impregnated with liquid flavor mixture prior to compression.

Alternatively, flavor may be added to the whole compressed chewing gum tablet by impregnation of the tablet with liquid flavor mixture.

It has been realized according to provisions of the present invention that flavor molecules are able to migrate into the core material. Through the impregnation of the chewing gum core with flavor it is possible to increase the amount of flavor in the core material to an extent which may not have been possible by prior art techniques without compromising the processability of the chewing gum core material during the manufacturing of the chewing gum core.

The amount of flavor applicable to a chewing gum core may be limited due to process demands. Adding flavor to the gum base comprised in the chewing gum core may negatively affect the further processing of the chewing gum core material. The softening of the core material by flavor, which has been a problem in prior art, will still occur by using the method according to the present invention. However, the softening will in part happen after final processing of the chewing gum, when further migration of flavor into the inner parts of the chewing gum core will take place. Therefore the problems related to softening may be avoided.

The mentioned migration of flavor molecules after the impregnation of the core material with liquid flavor mixture may be facilitated by appropriate choices of both core material and type of liquid flavor mixture.

An impregnation of the chewing gum core is characterized by that at least a part of the liquid flavor mixture will migrate into the inner parts of the chewing gum core with the passing of time. The time that is needed for a part of the flavor mixture to migrate into the inner parts of the core may depend on a number of different factors such as which chewing gum materials are used, the composition of the liquid flavor mixture and temperature and moisture conditions.

According to the first aspect of the invention said chewing gum core comprises at least a part of said active pharmaceutical ingredient.

In some embodiments of the invention a part of the active pharmaceutical ingredients are present in the chewing gum core and the remaining part of the active pharmaceutical ingredients are positioned in other parts of the chewing gum which may e.g. be in the coating.

In further embodiments of the invention the chewing gum core may comprise essentially all of the active pharmaceutical ingredients present in the chewing gum.

In an embodiment of the invention said optional coating comprises at least a part of said active pharmaceutical ingredient.

In some embodiments of the invention a coating of the chewing gum may comprise a part of the active pharmaceutical ingredient. In further embodiments of the invention the coating of the chewing gum may comprise essentially all of the active pharmaceutical ingredients present in the chewing gum.

In an embodiment of the invention said active pharmaceutical ingredient is nicotine.

In an embodiment of the invention said impregnation with said liquid flavor mixture is performed using multiple doses.

It may be advantageous to add flavor to the chewing gum core through impregnation with multiple doses. Hereby it may be possible to adjust the amount of flavor impregnated onto the chewing gum core through adaption of the number of doses of liquid flavor mixture used to impregnate the chewing gum core.

The impregnation with said liquid flavor mixture may be performed by using multiple doses such as 5, 10, 20, 50 doses or any other value depending on the specific liquid flavor mixture and the desired amount of flavor, and optionally other components comprised in the liquid flavor mixture, in the chewing gum core. However, in some embodiments and for some flavors impregnation with a single dose may be sufficient

Compared to processes in which flavor is incorporated into coatings, the impregnation method of the present invention may save considerable amounts of flavor and suspension, because the coating pan stays clean and a new dose of liquid flavor mixture will resolve residues, less material is lost and less cleaning is needed. In an ordinary coating process coating material may build up on the surfaces of the coating equipement and the flavor contained in the incrustation will be lost in the cleaning procedures.

Thus, shorter process times may be realized as well, since the need for cleaning the equipement is diminished.

Furthermore, some flavor components as for example cinnamon, have anti-bacterial capacity. Use of the impregnation method of the present invention with concentrated flavors may therefore help to diminish bacterial growth in the coating equipement.

In an embodiment of the invention each of said doses comprises 0.05 to 2.0 mg of liquid flavor mixture pr. chewing gum core.

In an embodiment of the invention each of said doses comprises 2.0 to 5.0 mg of liquid flavor mixture pr. chewing gum core.

In an embodiment of the invention each of said doses comprises 5.0 to 50 mg of liquid flavor mixture pr. chewing gum core.

The amount of liquid flavor mixture impregnated onto the chewing gum core pr.dose may vary depending on factors such as gum base composition, composition of the liquid flavor mixture and the use of impregnation aids.

In an embodiment of the invention said liquid flavor mixture comprises flavor in an amount of above 20, preferably above 40, most preferably above 60% by weight of the liquid flavor mixture.

Said liquid flavor mixture may have a viscosity in the range of 0.1 mPa·s to 1000 mPa·s at 25°C.

A wide range of viscosities of the liquid flavor mixture may be applicable in the impregnation step.

In an embodiment of the invention said optional coating is selected from the group consisting of a hard coating, a soft coating, a film coating, a gel coating or any combination thereof.

It may, in some cases, be beneficial to apply a coating onto the chewing gum after the impregnation with the liquid flavor mixture. Possible coatings may comprise any coating known in the art. The optional coating may comprise flavor. The impregnation method of the present invention does not impose any restraints on the optional coating as to flavor content or other coating ingredients known in the art.

Said optional coating may be a film coating.

Said optional coating may be a hard coating.

Said optional coating may be a combination of a film coating and a hard coating.

Said optional coating may comprise polymer.

Polymer containing coatings may be used for specific purposes such as chewing gum core protection or to comprise flavor and sweetener in a polymer matrix placed as a distinct layer on the chewing gum core.

In an embodiment of the invention said liquid flavor mixture comprises at least two different flavors.

The taste of a chewing gum may be a result of multiple flavors and varying release characteristics of those flavors. By impregnation of the chewing gum core it is possible to dose more than one flavor onto the chewing gum core in the same impregnation step, because the liquid flavor mixture may comprise two or more different flavors. The affinity of the chewing gum core for those different flavors may also be different, thereby influencing the flavor release characteristics of the chewing gum. Alternatively, an impregnation method comprising multiple steps may be used, for example alternating dosages of different liquid flavor mixtures.

In an embodiment of the invention said liquid flavor mixture comprises flavor selected from the group consisting of cinnamon, wintergreen, spearmint, orange and lemon.

In an embodiment of the invention said liquid flavor mixture comprises softener, emulsifier, high intensity sweetener, anti-oxidants and/or any combinations thereof. Many flavors and other ingredients may have a softening effect on the polymers comprised in the chewing gum core.

The softening may be a problem during chewing gum manufacturing in that the processing of the chewing gum core material may be obstructed, if the chewing gum core material gets too soft.

The impregnation with liquid flavor mixture having a softening effect allows at least part of the softening to take place after final processing of the chewing gum core material, thereby positively affecting the chewing gum manufacturing process. This softening effect may stem from the flavor possessing a softening effect itself or from other ingredients in the liquid flavor mixture, which have a softening effect on the polymers comprised in the chewing gum core.

Any substance having some affinity for the chewing gum core material may advantageously be added to the chewing gum core by impregnation. Distinct processing advantages and superior chewing gum compositions may be achieved by using one or more doses of liquid flavor mixture comprising chewing gum ingredients with affinity for the chewing gum core material.

In an embodiment of the invention said chewing gum core comprises flavor prior to impregnation.

A certain amount of flavors may be comprised in the chewing gum core prior to impregnation.

Further amounts of flavor may then be added to the chewing gum core by supplementing the flavor in the chewing gum core with one or more impregnation doses of liquid flavor mixture.

Said chewing gum core may be of a standard composition approved for use in pharmaceutical chewing gum.

It may be advantageous to add flavor to a pharmaceutical grade chewing gum core through impregnation steps. A liquid flavor mixture may be dosed onto a chewing gum core of standardized composition, thereby adding more flavor to the chewing gum core. The development time needed for the introduction of new flavors in connection with standardized core compositions may be considerably reduced by using the method of impregnation with liquid flavor mixture.

Said chewing gum core may be a conventional chewing gum core.

In an embodiment of the invention said chewing gum core is compressed.

Compression techniques to produce chewing gum are well known in the art. In some embodiments of the invention, flavor may be added to a compressed chewing gum by impregnation in order to increase the flavor content as described herein.

Said chewing gum core may be at least partly covered with powder prior to and/or during impregnation.

The powder may comprise flavor, process aids, active ingredients or any desired compound having the form of a powder.

In an embodiment of the invention said chewing gum core is at least partly covered with powdery process aids prior to and/or during impregnation.

In some embodiments of the invention powder may be added onto the chewing gum core to facilitate the processing of chewing gum core material. Liquid flavor mixture may be dosed onto chewing gum core covered with such process aid powder or residues thereof without seriously affecting the final outcome of the impregnation, that is, the addition of flavor to the chewing gum core. Alternating dosages of powder and liquid flqvor mixture may facilitate the impregnation process.

In an embodiment of the invention said powdery process aid is selected from the group consisting of polyol sugars such as maltitol, talc, high intensity sweeteners, calcium carbonate, solid flavor and combinations thereof.

The impregnation aids may help to initially absorb the liquid flavor mixture and may mediate the transfer of liquid flavor mixture components to the chewing gum core.

Said liquid flavor mixture may be a liquid at least in a range between 5-50°C.

According to provisions of the invention the liquid flavor mixture may be dosed onto the chewing gum core at ambient temperature. Therefore, in some embodiments of the invention it may be necessary that the liquid flavor mixture is a liquid in a temperature interval around typical ambient temperatures.

Said liquid flavor mixture may be a liquid at least in a range between 10 - 40°C.

Said optional coatings may be applied after the last dose of liquid flavor mixture is impregnated onto the chewing gum core.

The optional coating process is not affected by the impregnation prior to any optional coating. Optional coatings may be applied immediately after the last dose of liquid flavor mixture or, alternatively, after some time, depending on what is economical and practical in the production line.

Said liquid flavor mixture may comprise at least one solvent.

In some embodiments of the invention, thinning of flavor with one or more solvents may facilitate the process of impregnation. At least part of the solvents may evaporate from the chewing gum core after impregnation leaving the other flavor mixture components to migrate into the chewing gum core. The evaporation may be facilitated by blowing air around the chewing gum cores after final impregnation or even in between several impregnation steps.

Said liquid flavor mixture may be essentially solvent free.

In some embodiments of the invention, concentrated liquid flavor mixtures without solvents may be used for impregnation. Especially, this will be advantageous when the liquid flavor mixture has a suitable viscosity. With concentrated liquid flavor mixture, the number of doses necessary to achieve a certain concentration of flavor in the chewing gum core may be diminished.

Said at least one solvent may comprise water.

Some types of liquid flavor mixture components may be soluble in water, making it a suitable solvent in the impregnation process.

Said at least one solvent may comprise organic liquid.

Addition of organic solvent to a liquid flavor mixture may affect certain aspects of the impregnation process such as distribution of flavor just after impregnation and the viscosity of the liquid flavor mixture.

Said impregnation may comprise at least one step of spraying said liquid flavor mixture onto said chewing gum core.

A preferred method of dosing the liquid flavor mixture onto the chewing gum core is by spraying. The atomizing of liquid flavor mixture into droplets may be achieved through pressure, atomizing air or combinations thereof or any other means providing droplets of liquid flavor that can be passed onto the chewing gum core. Said impregnation may comprise at least one step of dipping said chewing gum core into said liquid flavor mixture.

Another method of dosing the liquid flavor mixture onto the chewing gum core is by dipping the chewing gum core into the liquid flavor mixture in a dipping process. Excess liquid flavor mixture for each step of dipping may run off the chewing gum core after the dipping leaving a desired amount of flavor behind which can migrate into the chewing gum core.

Said impregnation may comprise at least one step of rolling said liquid flavor mixture onto said chewing gum core.

Another method of dosing the liquid flavor mixture onto the chewing gum core for impregnation is by rolling the liquid flavor mixture onto the chewing gum core with dedicated rollers, typically picking up liquid flavor mixture from a reservoir. Desired amounts of flavor can be dosed onto the chewing gum core by adjusting for example the viscosity of liquid flavor mixture, the shape of the chewing gum core, type of roller and so on.

Said impregnation may be carried out at elevated pressures.

According to provisions of the invention, the impregnation step may be facilitated by process pressures higher than 1 atm. In some embodiments a pressure of above 1.5 atm. may be applied.

A method of prolonging the release of flavor from a chewing gum comprising a chewing gum core,
said chewing gum comprising active pharmaceutical ingredients, and
said method of prolonging the release of flavor from a chewing gum comprising the steps of:
- providing a chewing gum core comprising gum base and
- impregnating said chewing gum core by adding at least one dose of liquid flavor mixture onto the chewing gum core prior to any optional coating of the chewing gum core,
wherein at least the flavor in the liquid flavor mixture has affinity for the gum base in the chewing gum core, wherein said chewing gum core comprises at least a part of said active pharmaceutical ingredient.

It may be advantageous to control the release of flavor from a chewing gum and especially to prolong the release of flavor. According to the provisions of the invention, a high load of flavor into the chewing gum core may be achieved through the impregnation of the chewing gum core with liquid flavor mixture. This may lead to a chewing gum with advantageous slow flavor release from the chewing gum core, rendering a pleasant taste for prolonged periods.

Moreover, the invention relates to a method of adding flavor, preferably according to any of claims 1-13, whereby the release of flavor from a chewing gum is adjusted by storing said chewing gum under controlled conditions for at least 1 week, said controlled conditions comprises a temperature between 5-30°C, preferably between 10-25°C.

In advantageous embodiments of the invention, storing impregnated chewing gum for a period of time may considerably affect the release of flavor from the chewing gum. This is believed to be, at least in part, a result of flavor migration from the outer parts of the chewing gum core towards the central parts, whereby a more even flavor distribution in the chewing gum core may be reached. Upon chewing the gum, the flavor is released more slowly from the stored chewing gum when compared to a freshly made chewing gum.

A chewing gum with flavor added according to the provisions of the invention may be unique in comparison to prior art chewing gum in that very high loads of flavor in the chewing gum core may be achieved.

A method of adding flavor to at least one chewing gum granule
said method comprising the steps of:
- providing at least one chewing gum granule comprising gum base
- impregnating said at least one chewing gum granule by adding at least one dose of liquid flavor mixture onto said at least one chewing gum granule,
wherein the flavor in the liquid flavor mixture has affinity for the gum base in the chewing gum core.

An amount of flavor may be added to the individual granules by an impregnation as described herein prior to compression.

A compressed chewing gum comprising granules, wherein at least part of said granules has been impregnated by at least one dose of liquid flavor mixture.

The invention also relates to a chewing gum, wherein there is an inhomogeneous distribution of flavor in the chewing gum core and wherein the concentration of flavor is highest in the peripheral areas of the chewing gum core, wherein the inhomogeneous distribution of flavor provides improved dimensional stability to the chewing gum core, said chewing gum being obtainable by a process wherein the flavor is added by the method of any of the claims 1 - 14.

The invention furthermore relates to a chewing gum, wherein said inhomogeneous distribution of flavor is obtained after adding at least one dose of liquid flavor mixture onto the chewing gum core but prior to any optional coating of the chewing gum core.

Moreover, the invention relates to a method of adding flavor according to claim 1, wherein the chewing gum core exhibits improved dimensional stability for further processing.

The peripheral volume or periphery of the chewing gum core is also understood as the border volume, the edge, the outer parts, or the rim of the chewing gum core. Resulting from the impregnation of the chewing gum core by adding liquid flavor mixture onto the chewing gum core, it has been obtained that the concentration of flavor in the peripheral volume of the chewing gum core is higher than the concentration of flavor in the central volume of the chewing gum core. Hereby, advantageous possibilities of providing very tasteful chewing gum have been obtained and the softening of the chewing gum core due to components in the liquid flavor mixture is confined to the peripheral volume of the chewing gum core, facilitating the further processing of the chewing gum core.

One of the advantages obtained according to provisions of the invention is that a high content of flavor can be added to the chewing gum without compromising the dimensional stability of the chewing gum core. It has surprisingly been found that it is possible to increase the amount of flavor and improve the taste of the chewing gum significantly through a simple and cost-effective method involving impregnating the chewing gum cores with liquid flavor mixture. According to provisions of the present invention it may be possible to obtain an improved dimensional stability of chewing gum cores during production.

The softening effect of some of the liquid flavor mixture components on the chewing gum core is postponed until after the impregnation step. Because the migration of liquid flavor mixture components into the chewing gum core may be a slow process, it may be possible to finalize the chewing gum production based on the impregnated chewing gum cores before the softening effect is fully effective. The dimensions of the chewing gum core may thus be preserved in the finalizing process steps (e.g. coating, packaging) despite comparatively high amounts of e.g. flavor in the chewing gum core.

### DETAILED DESCRIPTION

With the present invention, as described in the following, a method of adding flavor to a chewing gum core comprising active pharmaceutical ingredients is provided.

It is obtained that flavor can be added to the chewing gum core after processing of the chewing gum core. Thereby the total amount of flavor in the chewing gum core can be optimized, the flavor release profile can be adjusted advantageously and the processing of the chewing gum core material can be simplified.

AI (AI: Active ingredients) as used herein is used to cover e.g. API (API: active pharmaceutical ingredients) and enhancers.

In the present context, a chewing gum core is simply the part of the chewing gum comprising gum base. Chewing gum core is not to be confused with a liquid or solid center fill and chewing gum core does not, in the present context, necessarily indicate a geometrical position in the middle of a chewing gum.

Chewing gum of the present invention typically comprises a water-soluble portion, a water-insoluble chewable gum base portion and flavoring agents. The water-soluble portion dissipates with a portion of the flavoring agent over a period of time during chewing. The gum base portion is retained in the mouth throughout the chew. The term chewing gum refers to both a chewing and bubble type gum in its general sense.

The gum base is the masticatory substance of the chewing gum, which imparts the chew characteristics to the final product. The gum base typically defines the release profile of flavors and sweeteners and plays a significant role in the gum product.

The gum base may be processed into granules and together with other ingredients be compressed into a compressed chewing gum tablet according to methods known in the art.

A liquid flavor mixture is impregnated onto the chewing gum core. At least one of the components of the liquid flavor mixture has affinity for the gum base comprised in the chewing gum core thereby migrating into the chewing gum core. The insoluble portion of the gum typically may contain any combination of elastomers, vinyl polymers, elastomer plasticizers, waxes, softeners, fillers and other optional ingredients such as colorants and antioxidants.

The elastomer compounds may be of natural origin or of synthetic origin, for example synthetic polyesters.

It is noted that the gum base may also include components typically referred to as chewing gum ingredients.

The chewing gum may, according to embodiments of the invention, comprise conventionally non-biodegradable polymers, such as natural resins, synthetic resins and/or synthetic or natural elastomers.

At least a part of the polymers of the chewing gum may be biodegradable.

The chewing gum may comprise combinations of biodegradable polymers and polymers generally regarded as non-biodegradable, such as natural resins, synthetic resins and/or synthetic/natural elastomers.

Said natural resin may comprise terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

Materials to be used for encapsulation methods may e.g. include Gelatine, Wheat protein, Soya protein, Sodium caseinate, Caseine, Gum arabic, modified starch, Hydrolyzed starches (maltodextrines), Alginates, Pectin, Carregeenan, Xanthan gum, Locus bean gum, Chitosan, Bees wax, Candelilla wax, Carnauba wax, Hydrogenated vegetable oils, Zein and/or Sucrose.

Examples of generally non-biodegradable synthetic resins include polyvinyl acetate, vinyl acetate-vinyl laurate copolymers and mixtures thereof. Examples of non-biodegradable synthetic elastomers include, but are not limited to, synthetic elastomers listed in Food and Drug Administration, CFR, Title 21, Section 172,615, Chewing Gum Base, Masticatory Substances, Synthetic Specifications, such as polyisobutylene e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

The elastomers (rubbers) employed in the gum base may vary depending upon various factors such as the type of gum base desired, the texture of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate, and the like, and mixtures thereof

It is common in the industry to combine in a gum base a synthetic elastomer having a high molecular weight with a synthetic elastomer having a low molecular weight. Examples of such combinations are polyisobutylene with styrene-butadiene, polyisobutylene with polyisoprene, polyisobutylene with isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Examples of natural resins are: Natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

The chewing gum according to embodiments of the invention may be provided with an outer coating.

The applicable hard coating may be selected from the group comprising sugar coating, a sugarless coating and a combination thereof. The hard coating may e.g. comprise 50 to 100% by weight of a polyol selected from the group consisting of sorbitol, maltitol, mannitol, xylitol, erythritol, lactitol and Isomalt and variations thereof. In an embodiment of the invention, the outer coating is an edible film comprising at least one component selected from the group consisting of an edible film-forming agent and a wax. The film-forming agent may e.g. be selected from the group comprising cellulose derivative, a modified starch, a dextrin, gelatine, shellac, gum arabic, zein, a vegetable gum, a synthetic polymer and any combination thereof. In an embodiment of the invention, the outer coating comprises at least one additive component selected from the group comprising of a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, an acid and an agent capable of accelerating the after-chewing degradation of the degradable polymer.

Generally, the ingredients may be mixed by first melting the gum base and adding it to the running mixer. Colors, active agents and/or emulsifiers may also be added at this time. A softener such as glycerin may also be added at this time, along with syrup and a portion of the bulking agent/sweetener. Further portions of the bulking agent/sweetener may then be added to the mixer. A flavoring agent may be added with the final portion of the bulking agent/sweetener. A high-intensity sweetener may be added after the final portion of bulking agent and optional flavor has been added.

The entire mixing procedure typically takes from five to sixty minutes, but longer mixing times may sometimes be required. Those skilled in the art will recognize that many variations of the above-described procedure may be followed. Including the one-step method described in US patent application 2004/0115305 hereby incorporated by reference. Chewing gums are formed by extrusion, compression and/or rolling and may be centre filled with liquids and/or solids in any form.

In further embodiments of the present invention, a chewing gum may also be provided with an outer coating, which may be a hard coating, a soft coating, a film coating, or a coating of any type that is known in the art, or a combination of such coatings. The coating may typically constitute 0.1 to 75% by weight of a coated chewing gum piece.

One preferred outer coating type is a hard coating, which term is including sugar coatings and sugar-free (or sugarless) coatings and combinations thereof. The object of hard coating is to obtain a sweet, crunchy layer, which is appreciated by the consumer and to protect the chewing gum core. In a typical process of providing the chewing gum cores with a protective sugar coating the chewing gum cores are successively treated in suitable coating equipment with aqueous solutions of crystallizable sugar such as sucrose or dextrose, which, depending on the stage of coating reached, may contain other functional ingredients, e.g. fillers, colors, etc.

The coating agent applied in a hard coating process may be a sugarless coating agent, e.g. a polyol including as examples sorbitol, maltitol, mannitol, xylitol, erythritol, lactitol and isomalt or e.g. a mono- di-saccharide including as example trehalose.

Or alternatively a sugar-free soft coating e.g. comprising alternately applying to the chewing gum cores a syrup of a polyol or a mono- di-saccharide, including as examples sorbitol, maltitol, mannitol, xylitol, erythritol, lactitol, isomalt and trehalose.

In further useful embodiments, a film coating is provided by film-forming agents such as a cellulose derivative, a modified starch, a dextrin, gelatine, zein, shellac, gum arabic, a vegetable gum, a synthetic polymer, etc. or a combination thereof.

The outer coating may comprise at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, and an acid.

A chewing gum core according to embodiments of the invention may have any form, shape or dimension that permits the chewing gum core to be coated using any conventional coating process.

The composition of gum base formulations can vary substantially depending on the particular product to be prepared and on the desired masticatory and other sensory characteristics of the final product. However, typical ranges of the above gum base components are: 5 to 80% by weight of elastomeric compounds, 5 to 80% by weight of elastomer plasticizers, 0 to 40% by weight of waxes, 5 to 35% by weight of softener, 0 to 50% by weight of filler, and 0 to 5% by weight of miscellaneous ingredients such as antioxidants, colorants, etc. The gum base may comprise about 5 to about 95% by weight of the chewing gum, more commonly; the gum base comprises 10 to about 60% by weight of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in gum base. This may be important when one wants to provide more elastomeric chain exposure to the alkanic chains of the waxes.

If desired, conventional elastomers or resins may be supplemented or substituted by biodegradable polymers.

Biodegradable polymers that may be used in the chewing gum of the present invention may be homopolymers, copolymers or terpolymers, including graft- and block-polymers.

Useful biodegradable polymers, which may be applied as gum base polymers in the chewing gum of the present invention, may generally be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed.

The usually preferred polyfunctional alcohols contain 2 to 100 carbon atoms as for instance polyglycols and polyglycerols.

Gum base polymers may both be resinous and elastomeric polymers.

In the polymerization of a gum base polymer for use in the chewing gum of the present invention, some applicable examples of alcohols, which may be employed as such or as derivatives thereof, include polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc.

Suitable examples of environmentally or biologically degradable chewing gum base polymers, which may be applied in accordance with the gum base of the present invention, include degradable polyesters, polycarbonates, polyester amides, polyesterurethanes, polyamides, prolamine, polypeptides, homopolymers of amino acids such as polylysine, and proteins including derivatives hereof such as e.g. protein hydrolysates including a zein hydrolysate.

Polyesters which may be applied in accordance with the gum base of the present invention may e.g. be as seen in EP 1 545 234 or EP 0 711 506 as incorporated herein by reference.

Further polymers which may be used in the gum base according to embodiments of the invention comprise:
enzymatically hydrolyzed zein, plasticized poly(D,L-lactic acid) and poly(D,L-lactic acid-co-glycolic acid), polyhydroxyalkanoates having side chain lengths of C₄ to C₃₀,
poly(lactic acid) copolymers selected from the group consisting of poly(lactic acid-dimer fatty acid-oxazoline) copolymers and poly(lactic acid-diol-urethane) copolymers,
at least one polyester, wherein the polyester includes monomers selected from the group consisting of lactic acid, lactide, glycolic acid, glycolide, citric acid, adipic acid, caprolactone, ethylene oxide, ethylene glycol, propylene oxide, and propylene glycol, and combinations thereof,
at least one polyester that is produced through a reaction of glycerol and at least one acid chosen from the group consisting of citric acid, fumaric acid, adipic acid, malic acid, succinic acid, suberic acid, sebacic acid, dodecanedioic acid, glucaric acid, glutamic acid, glutaric acid, azelaic acid, and tartaric acid,
at least one polyester that is produced through a reaction of at least one alcohol chosen from the group consisting of glycerol, propylene glycol, and 1,3 butylene diol, and at least one acid chosen from the group consisting of fumaric acid, adipic acid, malic acid, succinic acid, and tartaric acid, the polyester being end-capped with a monofunctional ingredient selected from the group consisting of alcohols, acids, chlorides, and esters,
and further such as can be found in e.g. US 6,773,730, US 6,613,363, US 6,194,008, US 5,580,590, US 6,858,238, US 6,017,566, US 6,013,287, and US 5,800,848, which are all hereby incorporated by reference.

The polyesters formed on the basis of di- or polyfunctional acids and di- or polyfunctional alcohols may be produced according to known methods, one of which includes US2007/043200, hereby incorporated by reference.

The prolamine may e.g. be selected from the group consisting of zein, corn gluten meal, wheat gluten, gliadin, glutenin and any combination thereof. Methods of providing such a polymer are disclosed in US2004/001903, hereby incorporated by reference.

Examples of such protein based compounds include but are not limited to prolamine, zein, corn gluten meal, wheat gluten, gliadin, glutenin and combinations thereof.

Such suitable biodegradable gum base polymers include polyesters, polycarbonates, polyesteramides, polyesterurethanes, polyamides, prolamine, and combinations thereof.

Carbonates may typically be co-polymerised with esters. Some typically preferred cyclic carbonates, which may be used as starting material, may e.g. comprise trimethylene carbonate, 2,2-dimethyltrimethylene carbonate, 2-methyltrimethylene carbonate, 3-methyltrimethylene carbonate, 2,3-dimethyltrimethylene carbonate, 2,4-dimethyltrimethylene carbonate, 2,3,4-trimethyltrimethylene carbonate, 2,3,3,4-tetramethyltrimethylene carbonate, etc.

In some embodiments, suitable polyesteramides can be constructed from monomers of the following groups: dialcohols, such as ethylene glycol, 1,4-butanediol, 1,3-propanediol, 1,6-hexanediol diethylene glycol and others; and/or dicarboxylic acid, such as oxalic acid, succinic acid, adipic acid and others, including those in the form of their respective esters (methyl, ethyl, etc.); and/or hydroxycarboxylic acids and lactones, such as caprolactone and others; and/or amino alcohols, such as ethanolamine, propanolamine, etc.; and/or cyclic lactams, such as .epsilon.-caprolactam or laurolactam, etc.; and/or .omega.-aminocarboxylic acids, such as aminocaproic acid, etc.; and/or mixtures (1:1 salts) of dicarboxylic acids such as adipic acid, succinic acid etc., and diamines such as hexamethyl enediamine, diaminobutane, etc.

In the case where the polymer mixture is based extensively on thermoplastic starch and an aromatic polyester, an aliphatic-aromatic copolyester, or a polyesteramide, it may be advantageous to add an aliphatic polyester or copolyester, such as polycaprolactone, for example, as a further component. As an example of this there may be mentioned a polymer mixture consisting of thermoplastic starch, at least one polyethylene terephthalate (PET) or a polyalkylene terephthalate, and polycaprolactone. Other examples of aliphatic polyesters or copolyesters are polylactic acid, polyhydroxybutyric acid, polyhydroxybutyric acid-hydroxyvaleric acid copolymer, and/or mixtures thereof.

Suitable polyesters may be obtained through polycondensation polymerisation or ring-opening polymerisation reactions. Some preferred polyesters include those polymerised from at least one carboxylic acid and at least one aliphatic di- or polyfunctional alcohols. The carboxylic acids may include aromatic dicarboxylic acids and aliphatic di- or polyfuncional carboxylic acids. In some preferred embodiments, the majority of the carboxylic acids are aliphatic.

Some of the preferred polyesters according to embodiments of the invention may e.g. be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed. Use of branched monomers suppresses the crystallinity of the polyester polymers. Mixing of dissimilar monomer units along the chain also suppresses crystallinity. To control the reaction and the molecular weight of the resulting polymer it is possible to stop the polymer chains by addition of monofunctional alcohols or acids and/or to utilize a stoichiometric imbalance between acid groups and alcohol groups or derivatives of either. Also the adding of long chain aliphatic carboxylic acids or aromatic monocarboxylic acids may be used to control the degree of branching in the polymer and conversely multifunctional monomers are sometimes used to create branching. Moreover, following the polymerization monofunctional compounds may be used to end cap the free hydroxyl and carboxyl groups.

Examples of aliphatic di- or polyfunctional carboxylic acids, which may be applied as monomers of suitable polyesters include oxalic, malonic, citric, succinic, malic, tartaric, fumaric, maleic, glutaric, glutamic, adipic, glucaric, pimelic, suberic, azelaic, sebacic, dodecanedioic acid, etc. Likewise, specific examples of aromatic polyfunctional carboxylic acids may be terephthalic, isophthalic, phthalic, trimellitic, pyromellitic and naphthalene 1,4-, 2,3-, 2,6-dicarboxylic acids and the like. Some preferred polyesters are disclosed in CA2523510, hereby included by reference.

Aliphatic dicarboxylic acids applied in the polyesters may be selected from aliphatic dicarboxylic acids having from 4 to 12 carbons, such as succinic acid, glutaric acid, 2-methylglutaric acid, 3-methylglutaric acid, 2,2-dimethylglutaric acid, adipic acid, pimelic acid, suberic acid, azelaic acid and sebacic acid, higher homologues and stereoisomers and mixtures thereof. Preferred aliphatic dicarboxylic acids in this embodiment are succinic acid, glutaric acid, adipic acid, pimelic acid, azelaic acid and sebacic acid, and mixtures thereof.

Aromatic dicarboxylic acids applied in the polyesters may contain two carboxyl groups which are bound to one aromatic system. Preferably, the aromatic system is carboaromatic, such as phenyl or naphthyl. In the case of polynuclear aromatics, the two carboxyl groups may be bound to the same ring or different rings. The aromatic system can also have one or more alkyl groups, for example methyl groups. The aromatic dicarboxylic acid is then generally selected from aromatic dicarboxylic acids having from 8 to 12 carbons, such as phthalic acid, isophthalic acid, terephthalic acid, 1,5- and 2,6-naphthalenedicarboxylic acid. Preferred aromatic dicarboxylic acids in this embodiment are terephthalic acid, isophthalic acid and phthalic acid and mixtures thereof.

Furthermore, some usually preferred polyfunctional alcohols suitable for preparing advantageous polyesters according to embodiments of the invention contain 2 to 100 carbon atoms as for instance polyglycols and polyglycerols. Suitable examples of alcohols, which may be employed in the polymerization process as such or as derivatives thereof, includes polyols such as ethylene glycol, 1,2-propanediol, 1,3-propanediol, 1,3-butanediol, 1,4-butanediol, 1,6-hexanediol, diethylene glycol, 1,4-cyclohexanediol, 1,4-cyclohexanedimethanol, neopentyl glycol, glycerol, trimethylolpropane, pentaerythritol, sorbitol, mannitol, etc. For the purpose of illustration and not limitation, some examples of alcohol derivatives include triacetin, glycerol palmitate, glycerol sebacate, glycerol adipate, tripropionin, etc.

Additionally, with regard to polyesters polymerized from alcohols or derivatives thereof and carboxylic acids or derivatives thereof, chain-stoppers sometimes used are monofunctional compounds. They are preferably either monohydroxy alcohols containing 1-20 carbon atoms or monocarboxylic acids containing 2-26 carbon atoms. General examples are medium or long-chain fatty alcohols or acids, and specific examples include monohydroxy alcohols such as methanol, ethanol, butanol, hexanol, octanol, etc., and lauryl alcohol, myristyl alcohol, cetyl alcohol, stearyl alcohol, stearic alcohol, etc., and monocarboxylic acids such as acetic, lauric, myristic, palmitic, stearic, arachidic, cerotic, dodecylenic, palmitoleic, oleic, linoleic, linolenic, erucic, benzoic, naphthoic acids and substituted napthoic acids, 1-methyl-2 naphthoic acid and 2-isopropyl-1-naphthoic acid, etc.

Typically, an acid catalyst or a transesterification catalyst may be used in such polyester polymerization processes, and non-limiting examples of those are the metal catalysts such as acetates of manganese, zinc, calcium, cobalt or magnesium, and antimony(III)oxide, germanium oxide or halide and tetraalkoxygermanium, titanium alkoxide, zinc or aluminum salts.

The polyesters can for example include copolymers containing any combination of the monomers lactic acid, lactide, glycolic acid, glycolide, citric acid, adipic acid, caprolactone, ethylene oxide, ethylene glycol, propylene oxide, propylene glycol and combinations thereof.

The polyesters may be obtained by the reaction between at least one dimer acid and at least one glycol or alcohol. Such glycols can include, for example, glycerin, propylene glycol, ethylene glycol, poly(ethylene glycol), poly(propylene glycol), poly(ethylene glycol-co-propylene glycol), while such alcohols can include, for example, methanol, ethanol, propanol, and butanol, and such dimer acids can include, for example, adipic acid and citric acid, etc.

Some specific examples of suitable polyesters include poly(lactic acid), polylactide, poly(glycolic acid), polyglycolide, poly(citric acid), polycaprolactone, polyhydroxyalkanoate, and combinations thereof.

Some suitable prolamines include zein, corn gluten meal, wheat gluten, gliadin, glutenin and combinations thereof. Moreover, blends of prolamine with polyester such as those disclosed in US 6,858,238, hereby included by reference, may be useful in chewing gum according to embodiments of the invention.

The chewing gum may include any component known in the chewing gum art. For example, the chewing gum may include elastomers, bulking agents, waxes, elastomer solvents, emulsifiers, plasticizers, fillers, and mixtures thereof.

The chewing gum according to embodiments of the invention may comprise coloring agents. According to an embodiment of the invention, the chewing gum may comprise color agents and whiteners such as FD&C-type dyes and lakes, fruit and vegetable extracts, titanium dioxide and combinations thereof.

Further useful chewing gum base components include antioxidants, e.g. butylated hydroxytoluene (BHT), butyl hydroxyanisol (BHA), propylgallate and tocopherols, and preservatives.

A gum base formulation may, in accordance with the present invention, comprise one or more softening agents e.g. sucrose esters including those disclosed in WO 00/25598, which is incorporated herein by reference, tallow, hydrogenated tallow, hydrogenated and partially hydrogenated vegetable oils, cocoa butter, degreased cocoa powder, glycerol monostearate, glyceryl triacetate, lecithin, mono-, di- and triglycerides, acetylated monoglycerides, lanolin, sodium stearate, potassium stearate, glyceryl lecithin, propylene glycol monostearate, glycerine, fatty acids (e.g. stearic, palmitic, oleic and linoleic acids) and combinations thereof. As used herein the term "softener" designates an ingredient, which softens the gum base or chewing gum formulation and encompasses waxes, fats, oils, emulsifiers, surfactants and solubilizers.

To soften the gum base further and to provide it with water-binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Useful emulsifiers can include, but are not limited to, glyceryl monostearate, propylene glycol monostearate, mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like and mixtures thereof are examples of conventionally used emulsifiers which can be added to the chewing gum base. In case of the presence of a biologically or active pharmaceutical ingredient as defined below, the formulation may comprise certain specific emulsifiers and/or solubilizers in order to disperse and release the active ingredient.

Waxes and fats are conventionally used for the adjustment of the texture and for softening of the chewing gum base when preparing chewing gum bases. In connection with the present invention, any conventionally used and suitable type of natural and synthetic wax and fat may be used, such as for instance rice bran wax, polyethylene wax, petroleum wax (refined paraffin and microcrystalline wax), sorbitan monostearate, tallow, propylene glycol, paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

A chewing gum base formulation may, if desired, include one or more fillers/texturisers including as examples, magnesium and calcium carbonate, sodium sulphate, ground limestone, silicate compounds such as magnesium and aluminum silicate, kaolin and clay, aluminum oxide, silicon oxide, talc, titanium oxide, mono-, di- and tri-calcium phosphates, cellulose polymers, such as wood, and combinations thereof.

In addition to a water insoluble gum base portion, a typical chewing gum includes a water soluble bulk portion and one or more flavoring agents. The water-soluble portion may include bulk sweeteners, high-intensity sweeteners, flavoring agents, softeners, emulsifiers, colors, acidulants, buffering agents, fillers, antioxidants, and other components that provide desired attributes.

Combinations of sugar and/or non-sugar sweeteners can be used in the chewing gum formulation processed in accordance with the invention. Additionally, the softener may also provide additional sweetness such as aqueous sugar or alditol solutions.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another chewing gum component such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0 to about 8% by weight, preferably 0.001 to about 5% by weight. When carriers used for encapsulation are included, the usage level of the encapsulated sweetener will be proportionately higher.

If a low-calorie gum is desired, a low-caloric bulking agent can be used. Examples of low caloric bulking agents include polydextrose, Raftilose, Raftilin, fructooligosaccharides (NutraFlora^{®}), palatinose oligosaccharides; guar gum hydrolysates (e.g. Sun Fiber^{®}) or indigestible dextrins (e.g. Fibersol^{®}). However, other low-calorie bulking agents can be used.

The chewing gum according to the present invention may contain aroma agents and flavoring agents including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

The chewing gum flavor may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors may also be used in the present chewing gum cores. As indicated above, the aroma agent may be used in quantities smaller than those conventionally used. The aroma agents and/or flavors may be used in the amount from 0.01 to about 30% by weight of the final product depending on the desired intensity of the aroma and/or flavor used. Preferably, the content of aroma/flavor is in the range of 0.2 to 5%, more preferably 0.5 to 3%, by weight of the total composition.

The flavoring agents may comprise natural and synthetic flavorings in the form of natural vegetable components, essential oils, essences, extracts, powders, including acids and other substances capable of affecting the taste profile.

The flavor may be used as taste masking in chewing gum comprising active ingredients, which by themselves have undesired taste or which alter the taste of the formulation.

Further chewing gum ingredients, which may be included in the chewing gum according to the present invention, include surfactants and/or solubilizers, especially when pharmaceutically or biologically active ingredients are present. As examples of types of surfactants to be used as solubilizers in a chewing gum composition according to embodiments of the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilizers can be used. Suitable solubilizers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilizers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubilizer may either be a single compound or a combination of several compounds. In the presence of an active ingredient, the chewing gum may preferably also comprise a carrier known in the art.

Active ingredients may advantageously be applied in a chewing gum according to the invention. Active ingredients generally refer to those ingredients that are included in a chewing gum composition for the desired end benefit they provide to the user. In some embodiments, active ingredients can include medicaments, nutrients, nutraceuticals, herbals, nutritional supplements, pharmaceuticals, drugs, and the like and combinations thereof. Moreover, in the present context, active ingredients may refer to flavor components, high intensity sweeteners or other taste establishing components.

Preferred API to be used in chewing gum according to embodiments of the invention are selected from the groups of antihistamines, anti-smoking agents, agents used for diabetes, decongestrants, peptides, pain-relieving agents, antacids, nausea-relieving agents, statines, and other.

Most preferred API according to embodiments of the invention are cetirizine, levo cetirizine, nicotine, nicotine polacrilex, nicotine in combination with alkaline agents, metformin, metformin HCL, phenylephrine, GLP-1, exenatide, MC-4 receptor antagonist, PPY(3-36), deca-peptide, KSL-W (acetate), fluor, and chlorhexidine.

Also preferred API according to embodiments of the invention are loratadine, desloratadine, nicotine bitartrate, nicotine in combination with caffeine, nicotine antagonists, combinations thereof or compounds comprising one or more of these, pseudoephedrine, flurbiprofen, paracetamol, acetylsalicylic acid, Ibuprofen, antacida, cimetidine, ranitidine, ondansetron, granisetron, metoclopramid, simvastatin, lovastatin, fluvastatin, acyclovir, benzydamin, rimonabant , varenicline, sildenafil, naltrexone, fluor in combination with fruit acids, derivatives, salts or isomers of chlorhexidine.

Some groups of suitable enhancers to e.g. enhance the uptake of API include bile salts, cetomacrogols, chelating agents, citrates, cyclodextrins, detergents, enamine derivatives, fatty acids, labrasol, lecithins, phospholipids, syntetic and natural surfactants, nonionic surfactants, cell envelope disordering compounds, solvents, steroidal detergents, chelators, solubilization agents, charge modifying agents, pH control agents, degradative enzyme inhibitors, mucolytic or mucus clearing agents.

Further groups of suitable enhancers include modulatory agents of epithelial junction physiology such as nitric oxide (NO) stimulators, chitosan, and chitosan derivatives; and vasodilator agents, selective transport-enhancing agents, stabilizing delivery vehicles, carriers, supports or complex- forming species with which exendins may be effectively combined, associated, contained, encapsulated or bound to stabilize an active agent for enhanced mucosal delivery; and membrane penetration-enhancing agents including surfactants, bile salts, phospholipid or fatty acid additives, mixed micelle, liposome, carrier, alcohol, enamine, NO donor compound, a long-chain amphipathic molecule, small hydrophobic penetration enhancer, sodium or a salicylic acid derivative, glycerol ester of acetoacetic acid, cyclodextrin or beta-cyclodextrin derivative, medium-chain fatty acid, chelating agent, amino acid or salt thereof, N-acetylamino acid or salt thereof, enzyme degradative to a selected membrane component, inhibitor of fatty acid synthesis, inhibitor of cholesterol synthesis, any combination of the membrane penetration enhancing agents.

Examples of enhancers suitable for application in chewing gum according to embodiments of the invention include cetylpyridinium chloride (CPC), benzalkonium chloride, sodium lauryl sulfate, polysorbate 80, Polysorbate 20, cetyltrimethylammonium bromide, laureth 9, sodium salicylate, sodium EDTA, EDTA, aprotinin, sodium taurocholate, saponins, bile salt derivatives, fatty acids, sucrose esters, azone emulsion, dextran sulphate, linoleic acid, labrafil, transcutol, urea, azone, nonionic surfactants, sulfoxides, sauric acid/PG, POE 23 lauryl ether, methoxysalicylate, dextran sulfate, methanol, ethanol, sodium cholate, Sodium taurocholate, Lysophosphatidyl choline, Alkylglycosides, polysorbates, Sorbitan esters, Poloxamer block copolymers, PEG-35 castor oil, PEG-40 hydrogenated castor oil, Caprocaproyl macrogol-8 glycerides, PEG-8 caprylic/capric, glycerides, Dioctyl sulfosuccinate, Polyethylene lauryl ether, Ethoxydiglycol, Propylene glycol, mono-di-caprylate, Glycerol monocaprylate, Glyceryl fatty acids (C.sub.8-C.sub.18) ethoxylated.

Oleic acid, Linoleic acid, Glyceryl caprylate/caprate, Glyceryl monooleate, Glyceryl monolaurate, Capryliccapric triglycerides, Ethoxylated nonylphenols, PEG-(8-50) stearates, Olive oil PEG-6, esters, Triolein PEG-6 esters, Lecithin, d-alpha tocopherol polyethylene glycol 1,000 succinate, Citric acid, Sodium citrate, BRIJ, Sodium laurate, 5-methoxysalicylic acid, Bile salts, Acetyl salicylate, ZOT, Docosahexaenoic acid, Alkylglycosides, Sodium glycocholate (GC-Na), Sodium taurocholate (TC-Na), EDTA, Choline salicylate, Sodium caprate (Cap-Na), N-lauryl-beta-D-maltopyranoside (LM), Diethyl maleate, Labrasol, Sodium salicylate, Mentol, Alkali metal alkyl sulphate, Sodium lauryl sulphate, Glycerin, Bile acid, Lecithin, phosphatidylcholine, phosphatidylserine, sphingomyelin, phophatidylethanolamine, cephalin, lysolecithin, Hyaluronic acid: alkalimetal salts, sodium, alkaline earth and aluminum, Octylphenoxypolyethoxyethanol, Glycolic acid, Lactic acid, Chamomile extract, Cucumber extract, Borage oil , Evening primrose oil, Polyglycerin, Lysine, Polylysine, Triolein, Monoolein, Monooleates, Monolaurates, Polydocanol alkyl ethers, Chenodeoxycholate, Deoxycholate, Glycocholic acid, Taurocholic acid, Glycodeoxycholic acid, Taurodeoxycholic acid, Sodium glycocholate, Phosphatidylcholine, Phosphatidylserine, Sphingomyelin, Phosphatidylethanolamine, Cephalin, Lysolecithin, Alkali metal hyaluronates, Chitosan, Poly-L-arginine, Alkyl glucoside, Saccharide alkyl ester, Fusidic acid derivatives, Sodium taurdihydrofusidate (STDHF), L-α-phosphatidylcholine Didecanoyl (DDPC), Nitroglycerine, nitropruside, NOC5 [3-(2-hydroxy-l-(methyl-ethyl)-2-nitrosohydrazino)-1- propanamine], NOC12 [iV-ethyl-2-(1-ethyl-hydroxy-2-nitrosohydrazino)-ethanamine, SNAP [S-nitroso-N-acetyl-DL-penicillamine, NORI, NOR4, deacylmethyl sulfoxide, azone, salicylamide, glyceryl-1,3-diacetoacetate, 1,2-isopropylideneglycerine-3-acetoacetate), Amino acids, Amino acid salts, monoaminocarboxlic acids, Glycine, alanine, phenylalanine, proline, hydroxyproline, hydroxyamino acids, serine, acidic amino acids, aspartic acid, Glutamic acid, Basic amino acids, Lysine, N-acetylamino acids, N-acetylalanine, N-acetylphenylalanine, TM-acetylserine, N-acetylglycine, N-acetyllysine, N-acetylglutamic acid, N-acetylproline, N-acetylhydroxyproline , lactic acid, malic acid and citric acid and alkali metal salts thereof, pyrrolidonecarboxylic acids, alkylpyrrolidonecarboxylic acid esters, N-alkylpyrrolidones, proline acyl esters, sodium lauryl phosphate, sodium lauryl sulphate, sodium oleyl phosphate, sodium myristyl sulphate, polyoxyethylene alkyl ethers, polyoxyethylene alkyl esters, and caproic acid, alkylsaccharide, fusidic acid, polyethylene glycol, cetyl alcohol, polyvinylpyrolidone, Polyvinyl alcohol, Lanolin alcohol, Sorbitan monooleate, Ethylene glycol tetraacetic acid, Bile acid conjugate with taurine, Cholanic acid and salts, Cyclodextran, Cyclodextrin, Cyclodextrin (beta), Hydroxypropyl-β-cyclodetran, Sulfobutylether-β-cyclodextran, Methyl-β-cyclodextrin, Chitosan glutamate, Chitosan acetate, Chitosan hydrochloride, Chitosan hydrolactate, 1-O-alkyl-2-hydroxy-sn-glycero-3-phosphocholine, 3-O-alkyl-2-acetoyl-sn-glycero-1-phosphocholine, 1-O-alkyl-2-O-acetyl-sn-glycero-3-phospho(N,N,N-trimethyl)hexanolamine, propylene glycol, tetradecylmaltoside (TDM), sucrose dedecanoate.

Examples of suitable mucoadhesives as enhancers according to embodiments of the invention include Carbopol 934+HPC, Maize + Carbopol 907, HPC (hydroxypropyl cellulose), Na-CMC, HPMC (hydroxypropylmethylcellulose), HEMA hydroxyethyl metacrylate, Carbopol 907 crosslinked with sucrose, Polyacrylic acids (PAA), Chitosans, Lectins, Polymetacrylate derivatives, Hyaluronic acid, P(AA-co-PEG) monomethylether monomethacrylate, PAA-PVP (Poly acrylic acid-poly vinyl pyrrilidone), PVP-PEG, methylcellulose, N-Trimethyl Chitosans, PDMAEMA, poly(dimethyl-aminoethyl methacrylate), HEC Hydroxethyl Cellulose, Carbomer 940, Carbomer 971, Polyethylene Oxide, Dextrin, Poly(Methyl Vinyl Ether/Maleic Anhydride), Polycarbophil that is polymers of acrylic acid crosslinked with divinyl glycol, PVP (PVP: Poly vinyl pyrrilidone), Agar, Tragacanth, Sodium Alginate, Karaya gum, MEC, HPC (HPC: Hydroxy propyl cellulose), Lectins, AB Block copolymer of oligo (methyl methacrylate) and PAA, Polymers with thiol groups, Spheromers, Thiomers, Alginic acid sodium salt, Carbopol 974P (Carbomer), EC (EC: Etylcellulose), CMC (CMC: Carboxymethyl cellulose), Dextran, Guar Gum, Pectins, Starch, Gelatin, Casein, Acrylic acid polymers, Polymers of acrylic acid esters, Acrylic acid copolymers, Vinyl polymers, Vinyl copolymers, Polymers of Vinyl alcohols, Alcoxy polymers, polyethylene oxide polymers, and polyethers.

Some groups of suitable enhancers include solubilization agents; charge modifying agents; pH control agents; degradative enzyme inhibitors; modulatory agents of epithelial junction physiology, such as nitric oxide (NO) stimulators, chitosan, or chitosan derivatives; vasodilator agents; selective transport-enhancing agents; stabilizing delivery vehicles, carriers, supports or complex- forming species with which exendin(s) is/are effectively combined, associated, contained, encapsulated or bound to stabilize the active agent for enhanced mucosal delivery; small hydrophilic penetration enhancers; emulsifiers, mucolytic or mucus clearing agents; membrane penetration-enhancing agents such as e.g., (i) a surfactant, (ii) a bile salt, (iii) a phospholipid or fatty acid additive, mixed micelle, liposome, or carrier, (iv) an alcohol, (v) an enamine, (iv) an NO donor compound, (vii) a long-chain amphipathic molecule, (viii) a small hydrophobic penetration enhancer, (ix) sodium or a salicylic acid derivative, (x) a glycerol ester of acetoacetic acid, (xi) a cyclodextrin or beta-cyclodextrin derivative, (xii) a medium-chain fatty acid, (xiii) a chelating agent, (xiv) an amino acid or salt thereof, (xv) an N-acetylamino acid or salt thereof, (xvi) an enzyme degradative to a selected membrane component, (xvii) an inhibitor of fatty acid synthesis, (xviii) an inhibitor of cholesterol synthesis; or (xiv) any combination of the membrane penetration enhancing agents of (i)-(xviii)).

In various embodiments of the invention, exendin is combined with one, two, three, four or more of the mucosal delivery-enhancing agents recited above.

Some suitable enhancers for application according to the present invention include pH control agents selected from the group consisting of Acetic acid, Adipic acid, Citric acid, Fumaric acid, Glucono-δ-lactone, Gluconic acid, Lactic acid, Malic acid, Maleic acid, Tartaric acid, Succinic acid, Propionic acid, Ascorbic acid, Phosphoric acid, Sodium orthophosphate, Potassium orthophosphate, Calcium orthophosphate, Sodium diphosphate, Potassium diphosphate, Calcium diphosphate, Pentasodium triphosphate, Pentapotassium triphosphate, Sodium polyphosphate, Potassium polyphosphate, Carbonic acid, Sodium carbonate, Sodium bicarbonate, Potasium carbonate, Calcium carbonate, Magnesium carbonate, Magnesium oxide, or any combination thereof.

The suitable pH control agents suitable according to the present invention include buffers.

Examples of dry binders to be used according to embodiments of the invention include mikro-crystalline cellulose (MCC), silicified micro-crystalline cellulose (SMCC), spray dried lactose, fast flow lactose, anhydrous lactose, sucrose, mannitol, mannitol EZ, dextrose, fructose, sorbitol, povidone, copovidone, dicalcium phosphate (DCP), starch (corn, potato and rice), pre-gelatinized starch, or combinations thereof. A number of filler materials may furthermore be used as dry binders.

Active ingredients may be classified according to The Anatomical Therapeutic Chemical (ATC) classification system, which is a system for classification of medicinal products according to their primary constituent and to the organ or system on which they act and their chemical, pharmacological and therapeutic properties.

The first level of the ATC is split into 14 main groups based on the anatomical group:
A: Alimentary tract and metabolism
B: Blood and blood forming organs
C: Cardiovascular system
D: Dermatologicals
G: Genito urinary system and sex hormones
H: Systemic hormonal preparations, excl. sex hormones and insulins
J: Antiinfectives for systemic use
L: Antineoplastic and immunomodulating agents
M: Musculo-skeletal system
N: Nervous system
P: Antiparasitic products, insecticides and repellents
R: Respiratory system
S: Sensory organs
V: Various

Further subdivision is done into a second, third, fourth and fifth sub-group, which is based on the therapeutic main group, the therapeutic/pharmacological subgroup, the chemical/therapeutic/pharmacological subgroup, and the chemical substance subgroup respectively. In this sense each active ingredient has been given a unique ATC identification code indicating where the active ingredient may be useful.

However, as some active ingredients are useful in more than one area, some of the active ingredients mentioned in this document belong to two or more of the mentioned groups, e.g. phenylephrine, which has an ATC identification code in both C, R, and S, i.e. both C01CA06, R01AA04, R01AB01, R01BA03, S01FB01, and S01GA05 are ATC identification codes identifying phenylephrine.

The following list discloses examples of active ingredients which can be classified according to the ATC classification mentioned above and which are active ingredients which may be used in a chewing gum according to embodiments of the invention:
Ephedrine, Magaldrate, Pseudoephedrine, Sildenafil, Xylocaine, Benzalconium chloride, Caffeine, Phenylephrine, Amfepramone, Orlistat, Sibutramine, Acetaminophen, Aspirin, Aluminum amino acetate, Aluminum amino acetate in combination with Magnesium oxide, Aluminum oxide hydrate in combination with Magnesiumoxide, Calcium carbonate in combination with Magnesium hydroxide, Calciumcarbonate, Dihydroxy Aluminum sodium carbonate, Magnesiumoxide, Glitazones, Metformin, Chlorpromazine, Dimenhydrinat, Domperidone, Meclozine, Metoclopramide, Odansetron, Prednisolone, Promethazine, Acrivastine, Cetirizine, Cinnarizine, Clemastine, Cyclizine, Desloratadine, Dexchlorpheniramine, Dimenhydrinate, Ebastine, Fexofenadine, Ibuprofen, Levolevoproricin, Loratadine, Meclozine, Mizolastine, Promethazine, Miconazole, Vitamin B12, Folic acid, Ferro compounds, vitamin C, Chlorhexidine diacetate, Fluoride, Decapeptide KSL, Aluminum fluoride, Aminochelated calcium, Ammonium fluoride, Ammonium fluorosilicate, Ammonium monofluorphosphate, Calcium fluoride, Calcium gluconate, Calcium glycerophosphate, Calcium lactate, Calcium monofluorphosphate, Calciumcarbonate, Carbamide, Cetyl pyridinium chloride, Chlorhexidine, Chlorhexidine digluconate, Chlorhexidine Chloride, Chlorhexidine diacetate, CPP Caseine Phospho Peptide, Hexetedine, Octadecentyl Ammonium fluoride, Potasium fluorosilicate, Potassium Chloride, Potassium monofluorphosphate, Sodium bi carbonate, Sodium carbonate, Sodium fluoride, Sodium fluorosilicate, Sodium monofluorphosphate, Sodium tri polyphosphate, Stannous fluoride, Stearyl Trihydroxyethyl Propylenediamine Dihydrofluoride, Strontium chloride, Tetra potassium pyrophosphate, Tetra sodium pyrophosphate, Tripotassium orthophosphate, Trisodium orthophosphate, Alginic acid, Aluminum hydroxide, Sodium bicarbonate, Sildenafil, Tadalafil, Vardenafil, Yohimbine, Cimetidine, Nizatidine, Ranitidine, Acetylsalicylic acid, Clopidogrel, Acetylcysteine, Bromhexine, Codeine, Dextromethorphan, Diphenhydramine, Noscapine, Phenylpropanolamine, vitamin D, Simvastatin, Bisacodyl, Lactitol, Lactulose, Magnesium oxide, Sodium picosulfate, Senna glycosides, Benzocaine, Lidocaine, Tetracaine, Almotriptan, Eletriptan, Naratriptan, Rizatriptan, Sumatriptan, Zolmitriptan, Calcium, Chromium, Copper, Iodine, Iron, Magnesium, Manganese, Molybdenium, Phosphor, Selenium, Zinc, Nicotine, Nicotine bitartrate, Nicotine pftalate, Nicotine polacrilex, Nicotine sulphate, Nicotine tartrate, Nicotine citrate, Nicotine lactate, Chloramine, Hydrogenperoxide, Metronidazole, Triamcinolonacetonide, Benzethonium Chl., Cetyl pyrid. Chl., Chlorhexidine, Fluoride, Lidocaine, Amphotericin, Miconazole, Nystatin, Fish oil, Ginkgo Biloba, Ginseng, Ginger, Purple coneflower, Saw Palmetto, Cetirizine, Levocetirizine, Loratadine, Diclofenac, Flurbiprofen, Acrivastine Pseudoephedrine, Loratadine Pseudoephedrine, Glucosamine, hyaluronic acid, Decapeptide KSL-W, Decapeptide KSL, Resveratrol, Misoprostol, Bupropion, Nicotine, Ondansetron HCl, Esomeprazole, Lansoprazole, Omeprazole, Pantoprazole, Rabeprazole, Bacteria and the like, Loperamide, Simethicone, Acetylsalicylic acid and others, Sucralfate, Vitamin A, Vitamin B1, Vitamin B12, Vitamin B2, Vitamin B6, Biotin, Vitamin C, Vitamin D, Vitamin E, Folinic acid, Vitamin K, Niacin, Q10, Clotrimazole, Fluconazole, Itraconazole, Ketoconazole, Terbinafine, Allopurinol, Probenecid, Atorvastatin, Fluvastatin, Lovastatin, Nicotinic acid, Pravastatin, Rosuvastatin, Simvastatin, Pilocarpine, Naproxen, Alendronate, Etidronate, Raloxifene, Risedronate, Benzodiazepines, Disulfiram, Naltrexone, Buprenorphine, Codeine, Dextropropoxyphene, Fentanyl, Hydromorphone, Ketobemidone, Ketoprofen, Methadone, Morphine, Naproxen, Nicomorphine, Oxycodone, Pethidine, Tramadol, Amoxicillin, Ampicillin, Azithromycin, Ciprofloxacin, Clarithromycin, Doxycyclin, Erythromycin, Fusidic acid, Lymecycline, Metronidazole, Moxifloxacin, Ofloxacin, Oxytetracycline, Phenoxymethylpenicillin, Rifamycins, Roxithromycin, Sulfamethizole, Tetracycline, Trimethoprim, Vancomycin, Acarbose, Glibenclamide, Gliclazide, Glimepiride, Glipizide, Insulin, Repaglinide, Tolbutamide, Oseltamivir, Aciclovir, Famciclovir, Penciclovir, Valganciclovir, Amlopidine, Diltiazem, Felodipine, Nifedipine, Verapamil, Finasteride, Minoxidil, Cocaine, Buphrenorphin, Clonidine, Methadone, Naltrexone, Calciumantagonists, Clonidine, Ergotamine, β-blockers, Aceclofenac, Celecoxib, Dexiprofen, Etodolac, Indometacin, Ketoprofen, Ketorolac, Lornoxicam, Meloxicam, Nabumetone, Oiroxicam, Parecoxib, Phenylbutazone, Piroxicam, Tiaprofenic acid, Tolfenamic acid, Aripiprazole, Chlorpromazine, Chlorprothixene, Clozapine, Flupentixol, Fluphenazine, Haloperidol, Lithium carbonate, Lithium citrate, Melperone, Penfluridol, Periciazine, Perphenazine, Pimozide, Pipamperone, Prochlorperazine, Risperidone, Thioridizin, Fluconazole, Itraconazole, Ketoconazole, Voriconazole, Opium, Benzodiazepines, Hydroxine, Meprobamate, Phenothiazine, Aluminumaminoacetate, Esomeprazole, Famotidine, Magnesium oxide, Nizatide, Omeprazole, Pantoprazole, Fluconazole, Itraconazole, Ketoconazole, Metronidazole, Amphetamine, Atenolol, Bisoprolol fumarate, Metoprolol, Metropolol, Pindolol, Propranolol, Auranofin, and Bendazac.

Further examples of useful active ingredients include active ingredients selected from the therapeutical groups comprising: Analgesic, Anaestetic, Antipyretic, Anti allergic, Anti-arrytmic, Appetite suppressant, Antifungal, Anti-inflammatory, Broncho dilator, Cardiovascular drugs, Coronary dilator, Cerebral dilator, Peripheral vasodilator, Anti-infective, Psychotropic, Anti- manic, Stimulant, Antihistamine, Laxative, Decongestrant, Gastro-intestinal sedative, Sexual dysfunction agent, Desinfectants, Anti-diarrheal, Anti-anginal substance, Vasodilator, Anti-hypertensive agent, Vasoconstrictor, Migraine treating agent, Antibiotic, Tranquilizer, Ntipsychotic, Anti-tumor drug, Anticoagulant, Antithrombotic agent, Hypnotic, Sedative, Anti-emetic, Anti-, auseant, Anticonvulsant, Neuromuscular agent, Hyper and hypoglycaemic, Thyroid and antithyroid, Diuretic, Antispasmodic, Uterine relaxant, Anti-obesity agent, Anoretic, Spasnolytics, Anabolic agent, Erythropoietic agent, Anti-asthmatic, Expectorant, Cough suppressant, Mucolytic, Anti-uricemic agent, Dental vehicle, Breath freshener, Antacid, Anti-diuretc, Anti-flatulent, Betablokker, Teeth Whitener, Enzyme, Co-enzyme, Protein, Energy booster, Fiber, Probiotics, Prebiotics, Antimicrobial agent, NSAID, Anti-tussives, Decongestrants, Anti-histamines, Expectorants, Anti-diarrheals, Hydrogen antagonists, Proton pump inhibitors, General nonselective CNS depressants, General nonselective CNS stimulants, Selectively CNS function modyfying drugs, Antiparkinsonism, Narcotic-analgetics, Analgetic-antipyretics, Psychopharmacological drugs, and Sexual dysfunction agents.

Examples of useful active ingredients include: Casein glyco-macro-peptide (CGMP), Nicotine, Nicotine bitartrate, Nicotine sulphate, Nicotine tartrate, Nicotine pftalate, Nicotine lactate, Nicotinecitrate, Nicotine polacrilex, Triclosan, Cetyl pyridinium chloride, Domiphen bromide, Quarternary ammonium salts, Zinc components, Sanguinarine, Fluorides, Alexidine, Octonidine, EDTA, Aspirin, Acetaminophen, Ibuprofen, Ketoprofen, Diflunisal, Fenoprofen calcium, Naproxen, Tolmetin sodium, Indomethacin, Benzonatate, Caramiphen edisylate, Menthol, Dextromethorphan hydrobromide, Theobromine hydrochloride, Chlophendianol Hydrochloride, Pseudoephedrine Hydrochloride, Phenylephrine, Phenylpropanolamine, Pseudoephedrine sulphate, Brompheniramine maleate, Chlorpheniramine- maleate, Carbinoxamine maleate, Clemastine fumarate, Dexchlorpheniramine maleate, Dephenhydramine hydrochloride, Diphenpyralide hydrochloride, Azatadine maleate, Diphenhydramine citrate, Doxylamine succinate, Promethazine hydrochloride, Pyrilamine maleate, Tripellenamine citrate, Triprolidine hydrochloride, Acrivastine, Loratadine, Brompheniramine, Dexbrompheniamine, Guaifenesin, Ipecac, Potassium iodide, Terpin hydrate, Loperamide, Famotidine, Ranitidine, Omeprazole, Lansoprazole, Aliphatic alcohols, Barbiturates, Caffeine, Nicotine, Strychnine, Picrotoxin, Pentyenetetrazol, Phenyhydantoin, Phenobarbital, Primidone, Carbamazapine, Etoxsuximide, Methsuximide, Phensuximide, Trimethadione, Diazepam, Benzodiazepines, Phenacemide, Pheneturide, Acetazolamide, Sulthiame, Bromide, Levodopa, Amantadine, Morphine, Heroin, Hydromorphone, Metopon, Oxymorphone, Levophanol, Codeine, Hydrocodone, Xycodone, Nalorphine, Naloxone, Naltrexone, Salicylates, Phenylbutazone, Indomethacin, Phenacetin, Chlorpromazine, Methotrimeprazine, Haloperidol, Clozapine, Reserpine, Imipramine, Tranylcypromine, Phenelzine, Lithium, Sildenafil citrate, Tadalafil, and Vardenafil CL.

Examples of useful active ingredients include active ingredients selected from the groups of ace-inhibitors, antianginal drugs, anti- arrhythmias, anti-asthmatics, anti-cholesterolemics, analgesics, anesthetics, anticonvulsants, anti-depressants, antidiabetic agents, anti-diarrhea preparations, antidotes, anti-histamines, anti-hypertensive drugs, anti-inflammatory agents, anti-lipid agents, anti- manics, antinauseants, anti-stroke agents, anti-thyroid preparations, anti-tumor drugs, anti- viral agents, acne drugs, alkaloids, amino acid preparations, anti-tussives, anti- uricemic drugs, anti- viral drugs, anabolic preparations, systemic and non-systemic anti-infective agents, anti-neoplasties, antiparkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocriptine or nicotine, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, antinauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of active ingredients contemplated for use in the present invention can include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other drug active ingredients for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haldol™; non-steroidal antiinflammatories (NSAID's) such as ibuprofen, naproxen sodium, Voltaren™ and Lodine™, anti-histamines such as Claritin™, Hismanal™, Relafen™, and Tavist™; antiemetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium alumino silicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts. A variety of nutritional supplements may also be used as active ingredients including virtually any vitamin or mineral. For example, vitamin A, vitamin C, vitamin D, vitamin E, vitamin K, vitamin B6, vitamin B12, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used. Examples of nutritional supplements that can be used as active ingredients are set forth in U.S. Patent Application Publication Nos. 2003/0157213 A1, 2003/0206993 and 2003/0099741 A1 which are incorporated in their entirety herein by reference for all purposes. Various herbals may also be used as active ingredients such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Ginko Biloba, St. John's Wort, Evening Primrose Oil, Yohimbe Bark, Green Tea, Ma Huang, Maca, Bilberry, Lutein, and combinations thereof.

Especially when hydrophilic, encapsulation of the active ingredient will result in a delay in the release of the predominant amount of the active ingredient during consumption of a chewing gum that includes the encapsulated active ingredient (e.g., as part of a delivery system added as an ingredient to the chewing gum), in some embodiments, the release profile of the ingredient (e.g., the active ingredient) can be managed for a chewing gum by managing various characteristics of the ingredient, delivery system containing the ingredient, and/or the chewing gum containing the delivery system and/or how the delivery system is made. For example, characteristics may include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the compressible chewing gum, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

In some embodiments, the release profiles of one or more components of an effervescing system are managed for a chewing gum. The effervescent system may include one or more edible acids and one or more edible alkaline materials. The edible acid(s) and the edible alkaline material(s) may react together to generate effervescence. In some embodiments, the alkaline material(s) may be selected from, but is not limited to, alkali metal carbonates, alkali metal bicarbonates, alkaline earth metal carbonates, alkaline earth metal bicarbonates, and combinations thereof. The edible acid(s) may be selected from, but is not limited to, citric acid, phosphoric acid, tartaric acid, malic acid, ascorbic acid, and combinations thereof In some embodiments, an effervescing system may include one or more other ingredients such as, for example, carbon dioxide, oral care ingredients, flavorants, etc.

For examples of use of an effervescing system in a chewing gum, refer to U.S. Provisional Patent No. 60/618,222 filed October 13, 2004, and entitled "Effervescent Pressed Gum Tablet Compositions," the contents of which are incorporated herein by reference for all purposes. Other examples can be found in U.S. Patent No. 6,235,318, the contents of which are incorporated herein by reference for all purposes.

In some embodiments, the release profiles of one or more appetite suppressors are managed for a chewing gum. Appetite suppressors can be ingredients such as fiber and protein that function to depress the desire to consume food. Appetite suppressors can also include benzphetamine, diethylpropion, mazindol, phendimetrazine, phentermine, hoodia (P57), Olibra™, ephedra, caffeine and combinations thereof. Appetite suppressors are also known by the following trade names: Adipex™, Adipost™, Bontril™ PDM, Bontril™ Slow Release, Didrex™, Fastin™, Ionamin™, Mazanor™, Melfiat™, Obenix™, Phendiet™, Phendiet-105™, Phentercot™, Phentride™, Plegine™, Prelu-2™, Pro-Fast™, PT 105™, Sanorex™, Tenuate™, Sanorex™, Tenuate™, Tenuate Dospan™, Tepanil Ten-Tab™, Teramine™, and Zantryl™. These and other suitable appetite suppressors are further described in the following U.S. patents, all of which are incorporated in their entirety by reference hereto: U.S. 6,838,431 to Portman, U.S. 6,716,815 to Portman, U.S. 6,558,690 to Portman, U.S. 6,468,962 to Portman, U.S. 6,436,899 to Portman.

In some embodiments, the release profiles of one or more breath fresheners are managed for a chewing gum. Breath fresheners can include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments, breath fresheners can include but are not limited to zinc citrate, zinc acetate, zinc fluoride, zinc ammonium sulfate, zinc bromide, zinc iodide, zinc chloride, zinc nitrate, zinc flurosilicate, zinc gluconate, zinc tartarate, zinc succinate, zinc formate, zinc chromate, zinc phenol sulfonate, zinc dithionate, zinc sulfate, siliver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof. In some embodiments, the release profiles of probiotics can be managed for a compressible gum including, but not limited to lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names: Retsyn™, Actizol™, and Nutrazin™. Examples of malodor-controlling compositions are also included in U.S. Patent No. 5,300,305 to Stapler et al. and in U.S. Patent Application Publication Nos. 2003/0215417 and 2004/0081713 which are incorporated in their entirety herein by reference for all purposes.

In some embodiments, the release profiles of one or more dental care ingredients may be managed for a chewing gum. Such dental care ingredients (also known as oral care ingredients) may include but are not limited to tooth whiteners, stain removers, oral cleaning, bleaching agents, desensitizing agents, dental remineralization agents, antibacterial agents, anticaries agents, plaque acid buffering agents, surfactants and anticalculus agents. Non-limiting examples of such ingredients can include, hydrolytic agents including proteolytic enzymes, abrasives such as hydrated silica, calcium carbonate, sodium bicarbonate and alumina, other active stain-removing components such as surface-active agents, including, but not limited to anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed as tartar control ingredients. In some embodiments, dental care ingredients can also include tetrasodium pyrophosphate and sodium tri-polyphosphate, sodium bicarbonate, sodium acid pyrophosphate, sodium tripolyphosphate, xylitol, sodium hexametaphosphate. In some embodiments, peroxides such as carbamide peroxide, calcium peroxide, magnesium peroxide, sodium peroxide, hydrogen peroxide, and peroxydiphospate are included. In some embodiments, potassium nitrate and potassium citrate are included. Other examples can include casein glycomacropeptide, calcium casein peptone-calcium phosphate, casein phosphopeptides, casein phosphopeptide- amorphous calcium phosphate (CPP-ACP), and amorphous calcium phosphate. Still other examples can include papaine, krillase, pepsin, trypsin, lysozyme, dextranase, mutanase, glycoamylase, amylase, glucose oxidase, and combinations thereof. Further examples can include surfactants such as sodium stearate, sodium ricinoleate, and sodium lauryl sulfate surfactants for use in some embodiments to achieve increased prophylactic action and to render the dental care ingredients more cosmetically acceptable. Surfactants can preferably be detersive materials which impart to the composition detersive and foaming properties. Suitable examples of surfactants are water-soluble salts of higher fatty acid monoglyceride monosulfates, such as the sodium salt of the monosulfated monoglyceride of hydgrogenated coconut oil fatty acids, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine. In addition to surfactants, dental care ingredients can include antibacterial agents such as, but not limited.to, triclosan, chlorhexidine, zinc citrate, silver nitrate, copper, limonene, and cetyl pyridinium chloride. In some embodiments, additional anticaries agents can include fluoride ions or fluorine-providing components such as inorganic fluoride salts. In some embodiments, soluble alkali metal salts, for example, sodium fluoride, potassium fluoride, sodium fluorosilicate, ammonium fluorosilicate, sodium monofluorophosphate, as well as tin fluorides, such as stannous fluoride and stannous chloride can be included. In some embodiments, a fluorine-containing compound having a beneficial effect on the care and hygiene of the oral cavity, e.g., diminution of enamel solubility in acid and protection of the teeth against decay may also be included as an ingredient. Examples thereof include sodium fluoride, stannous fluoride, potassium fluoride, potassium stannous fluoride (SnF.sub.2 -KF), sodium hexafluorostannate, stannous chlorofluoride, sodium fluorozirconate, and sodium monofluorophosphate. In some embodiments, urea is included. Further examples are included in the following U.S. patents and U.S. published patent applications, the contents of all of which are incorporated in their entirety herein by reference for all purposes: U.S. Patent Nos. 5,227,154 to Reynolds, 5,378,131 to Greenberg, 6,846,500 to Luo et al, 6,733,818 to Luo et al., 6,696,044 to Luo et al., 6,685,916 to Holme et al., 6,485,739 to Luo et al., 6,479,071 to Holme et al., 6,471,945 to Luo et al., U.S. Patent Publication Nos. 20050025721 to Holme et al., 2005008732 to Gebreselassie et al., and 20040136928 to Holme et al.

In some embodiments, the release profiles of one or more flavor potentiators can be managed for a chewing gum. Flavor potentiators can consist of materials that may intensify, supplement, modify or enhance the taste and/or aroma perception of an original material without introducing a characteristic taste and/or aroma perception of their own. In some embodiments, potentiators designed to intensify, supplement, modify, or enhance the perception of flavor, sweetness, tartness, umami, kokumi, saltiness and combinations thereof can be included. In some embodiments, sweetness may be potentiated by the inclusion of monoammonium glycyrrhizinate, licorice glycyrrhizinates, citrus aurantium, maltol, ethyl maltol, vanilla, vanillin, and combinations thereof. In some embodiments, sugar acids, sodium chloride, potassium chloride, sodium acid sulfate, and combinations thereof may be included for flavor potentiation. In other examples, glutamates such as monosodium glutamate (MSG), monopotassium glutamate, hydrolyzed vegetable protein, hydrolyzed animal protein, yeast extract, and combinations thereof are included. Further examples can include glutathione, and nucleotides such as inosine monophosphate (IMP), disodium inosinate, xanthosine monophosphate, guanylate monophosphate (GMP), and combinations thereof. For bitterness blocking or taste masking, ingredients that interact with bitterness receptors to suppress bitterness or off tastes may be included. In some embodiments, adenosine monophosphate (AMP) can be included for bitterness suppression. Bitterness modification can also be accomplished by using sweetness or flavors with complementary bitter notes such as chocolate. Further examples of flavor potentiator compositions that impart kokumi are also included in U.S. Patent No. 5,679,397 to Kuroda et al, the entire contents of which are incorporated in its entirety herein by reference.

Typically, encapsulation of a flavor potentiator will result in a delay in the release of the predominant amount of the flavor potentiator during consumption of a chewing gum that includes the encapsulated flavor potentiator (e.g., as part of a delivery system added as an ingredient to the chewing gum composition). In some embodiments, the release profile of the ingredient (e.g., the flavor potentiator) can be managed for a chewing gum by managing various characteristics of the ingredient, delivery system containing the ingredient, and/or the compressible chewing gum containing the delivery system and/or how the delivery system is made.

In some embodiments, the release profiles of one or more acids may be managed for a chewing gum. Acids can include, but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof.

Typically, encapsulation of a food acid will result in a delay in the release of the predominant amount of the active ingredient during consumption of a compressible chewing gum that includes the encapsulated food acid (e.g., as part of a delivery system added as an ingredient to the chewing gum).

In some embodiments, the release profiles of one or more micronutrients can be managed for a chewing gum. Micronutrients can include materials that have an impact on the nutritional wellbeing of an organism even though the quantity required by the organism to have the desired effect is small relative to macronutrients such as protein, carbohydrate, and fat. Micronutrients can include, but are not limited to vitamins, minerals, enzymes, phytochemicals, antioxidants, and combinations thereof. In some embodiments, vitamins can include fat soluble vitamins such as vitamin A, vitamin D, vitamin E, and vitamin K and combinations thereof, in some embodiments, vitamins can include water soluble vitamins such as vitamin C (ascorbic acid), the B vitamins (thiamine or B1, riboflavoin or B2, niacin or B3, pyridoxine or B6, folic acid or B9, cyanocobalimin or B12, pantothenic acid, biotin), and combinations thereof.

In some embodiments, minerals can include but are not limited to sodium, magnesium, chromium, iodine, iron, manganese, calcium, copper, fluoride, potassium, phosphorous, molybdenum, selenium, zinc, and combinations thereof.

In some embodiments micronutrients can include but are not limited to L- carnitine, choline, coenzyme Q10, alpha-lipoic acid, omega-3 -fatty acids, pepsin, phytase, trypsin, lipases, proteases, cellulases and combinations thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, phytochemicals can include but are not limited to cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate, theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, sterols, stanols, stanol esters, sterol esters and combinations thereof.

Typically, encapsulation of the micronutrient will result in a delay in the release of the predominant amount of the active ingredient during consumption of a chewing gum that includes the encapsulated micronutrient (e.g., as part of a delivery system added as an ingredient to the chewing gum).

In some embodiments, the release profiles of one or more mouth moisteners can be managed for a compressible gum. Mouth moisteners can include, but are not limited to, saliva stimulators such as acids and salts and combinations thereof. In some embodiments, acids can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof Mouth moisteners can also include hydrocolloid materials that hydrate and may adhere to oral surface to provide a sensation of mouth moistening. Hydrocolloid materials can include naturally occurring materials such as plant exudates, seed gums, and seaweed extracts or they can be chemically modified materials such as cellulose, starch, or natural gum derivatives. In some embodiments, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, bacterial gums, and combinations thereof. Additionally, in some embodiments, modified natural gums such as propylene glycol alginate, carboxymethyl locust bean gum, low methoxyl pectin, and their combinations can be included. In some embodiments, modified celluloses can be included such as microcrystalline cellulose, carboxymethlcellulose (CMC), methylcellulose (MC), hydroxypropylmethylcellulose (HPMC), and hydroxypropylcellulose (MPC), and combinations thereof. Similarly, humectants which can provide a perception of mouth hydration can be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof. Typically, encapsulation of a mouth moistening agent will result in a delay in the release of the predominant amount of the active ingredient during consumption of a chewing gum that includes the encapsulated mouth moistening agent (e.g., as part of a delivery system added as an ingredient to the compressible chewing gum). In some embodiments, the release profile of the ingredient (e.g., the mouth moistening agent) can be managed for a compressible gum by managing various characteristics of the ingredient, delivery system containing the ingredient, and/or the compressible chewing gum containing the delivery system and/or how the delivery system is made.

In some embodiments, the release profiles of one or more ingredients that soothe the throat can be managed for a chewing gum. Throat soothing ingredients can include analgesics, anesthetics, demulcents, antiseptic, and combinations thereof. In some embodiments, analgesics/anesthetics can include menthol, phenol, hexylresorcinol, benzocaine, dyclonine hydrochloride, benzyl alcohol, salicyl alcohol, and combinations thereof. In some embodiments, demulcents can include but are not limited to slippery elm bark, pectin, gelatin, and combinations thereof. In some embodiments, antiseptic ingredients can include cetylpyridinium chloride, domiphen bromide, dequalinium chloride, and combinations thereof.

In some embodiments, antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof can be included.

In some embodiments, throat soothing agents such as honey, propolis, aloe vera, glycerine, menthol and combinations thereof can be included. In still other embodiments, cough suppressants can be included. Such cough suppressants can fall into two groups: those that alter the texture or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, antitussives can include, but are not limited to, the group consisting of codeine, dextromethorphan, dextrorphan, diphenhydramine, hydrocodone, noscapine, oxycodone, pentoxyverine and combinations thereof. In some embodiments, antihistamines can include, but are not limited to, acrivastine, azatadine, brompheniramine, chlo[phi]heniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof In some embodiments, non-sedating antihistamines can include, but are not limited to, astemizole, cetirizine, ebastine, fexofenadine, loratidine, terfenadine, and combinations thereof.

In some embodiments, expectorants can include, but are not limited to, ammonium chloride, guaifenesin, ipecac fluid extract, potassium iodide and combinations thereof. In some embodiments, mucolytics can include, but are not limited to, acetylcycsteine, ambroxol, bromhexine and combinations thereof. In some embodiments, analgesic, antipyretic and anti-inflammatory agents can include, but are not limited to, acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, local anesthetics can include, but are not limited to, lidocaine, benzocaine, phenol, dyclonine, benzonotate and mixtures thereof. In some embodiments nasal decongestants and ingredients that provide the perception of nasal clearing can be included. In some embodiments, nasal decongestants can include but are not limited to phenylpropanolamine, pseudoephedrine, ephedrine, phenylephrine, oxymetazoline, and combinations thereof. In some embodiments ingredients that provide a perception of nasal clearing can include but are not limited to menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, gums, flowers and other botanicals, resins, animal secretions, and synthetic aromatic materials.

Typically, encapsulation of a throat care agent will result in a delay in the release of the predominant amount of the active ingredient during consumption of a compressible chewing gum that includes the encapsulated throat care agent (e.g. as part of a delivery system added as an ingredient to the compressible chewing gum).

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof In some embodiments, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E161g), cryptoxanthin (E161c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These consist of the aluminum salts of FD&C dyes extended on an insoluble substrate of alumina hydrate. Additionally, in some embodiments, certified colors can be included as calcium salts.

Typically, encapsulation of a color will result in a delay in the release of the predominant amount of the active ingredient during consumption of a chewing gum that includes the encapsulated color (e.g., as part of a delivery system added as an ingredient to the compressible chewing gum).

In some embodiments, a delivery system or chewing gum may include two or more ingredients for which managed release from the compressible chewing gum during consumption of the compressible chewing gum is desired. In some embodiments, the ingredients may be encapsulated or otherwise included separately in different delivery systems. Alternatively, in some embodiments the ingredients may be encapsulated or otherwise included in the same delivery system. As another possibility, one or more of the ingredients may be free (e.g. unencapsulated) while one or more other ingredients may be encapsulated. A chewing gum may include a group of ingredients for which managed release of the group during consumption of the chewing gum is desired. Groups of two or more ingredients for which managed release from a chewing gum during consumption of the chewing gum may be desired include, but are not limited to: color and flavor, multiple flavors, multiple colors, cooling agent and flavor, warming agent and flavor, cooling agent and warming agent, cooling agent and high-intensity sweetener, warming agent and high-intensity sweetener, multiple cooling agents (e.g., WS-3 and WS-23, WS-3 and menthyl succinate), menthol and one or more cooling agents, menthol and one or more warming agents, multiple warming agents, high-intensity sweetener(s) and tooth whitening active(s), high-intensity sweetener(s) and breath-freshening active(s), an ingredient with some bitterness and a bitterness suppressor for the ingredient, multiple high-intensity sweeteners (e.g., acesulfame-k and aspartame), multiple tooth whitening active ingredients (e.g., an abrasive ingredient and an antimicrobial ingredient, a peroxide and a nitrate), a warming agent and a polyol, a cooling agent and a polyol, multiple polyols, a warming agent and micronutrient, a cooling agent and a micronutrient, a warming agent and a mouth moistening agent, a cooling agent and a mouth moistening agent, a warming agent and a throat care agent, a cooling agent and a throat care agent, a warming agent and a food acid, a cooling agent and food acid, a warming agent and an emulsifier/surfactant, a cooling agent and an emulsifier/surfactant, a warming agent and a color, a cooling agent and a color, a warming agent and a flavor potentiator, a cooling agent and a flavor potentiator, a warming agent with sweetness potentiator, a cooling agent with a sweetness potentiator, a warming agent and an appetite suppressant, a cooling agent and an appetite suppressant, a high-intensity sweetener and a flavor, a cooling agent and a teeth-whitening agent, a warming agent and a teeth-whitening agent, a warming agent and breath-freshening agent, a cooling agent and a breath-freshening agent, a cooling agent and an effervescing system, a warming agent and an effervescing system, a warming agent and an antimicrobial agent, a cooling agent and an antimicrobial agent, multiple anticalcums ingredients, multiple remineralization ingredients, multiple surfactants, remineralization ingredients with demineralization ingredients, acidic ingredients with acid buffering ingredients, anticalculus ingredients with antibacterial ingredients, remineralization ingredients with anticalculus ingredients, anticalculus ingredients with remineralization ingredients with antibacterial ingredients, surfactant ingredients with anticalculus ingredients, surfactant ingredients with antibacterial ingredients, surfactant ingredients with remineralization ingredients, surfactants with anticalculus ingredients with antibacterial ingredients, multiple types of vitamins or minerals, multiple micronutrients, multiple acids, multiple antimicrobial ingredients, multiple breath-freshening ingredients, breath-freshening ingredients and antimicrobial ingredients, multiple appetite suppressors, acids and bases that react to effervesce, a bitter compound with a high-intensity sweetener, a cooling agent and an appetite suppressant, a warming agent and an appetite suppressant, a high-intensity sweetener and an appetite suppressant, a high-intensity sweetener with an acid, a probiotic ingredient and a prebiotic ingredient, a vitamin and a mineral, a metabolic enhancement ingredient with a macronutrient, a metabolic enhancement ingredient with a micronutrient, an enzyme with a substrate, a high-intensity sweetener with a sweetness potentiator, a cooling compound with a cooling potentiator, a flavor with a flavor potentiator, a warming compound with a warming potentiator, a flavor with salt, a high-intensity sweetener with salt, an acid with salt, a cooling compound with salt, a warming compound with salt, a flavor with a surfactant, an astringent compound with an ingredient to provide a sensation of hydration, etc. In some embodiments, the multiple ingredients may be part of the same delivery system or may be part of different delivery systems. Different delivery systems may use the same or different encapsulating materials.

Typically, encapsulation of the multiple ingredients will result in a delay in the release of the predominant amount of the multiple ingredients during consumption of a compressible chewing gum that includes the encapsulated multiple ingredients (e.g. as part of a delivery system added as an ingredient to the compressible chewing gum). This may be particularly helpful in situations wherein separate encapsulation of the ingredients may cause them to release with different release profiles. For example, different high-intensity sweeteners may have different release profiles because they have different water solubilities or differences in other characteristics. Encapsulating them together may cause them to release more simultaneously.

In some embodiments, the release profile of the multiple ingredients can be managed for a chewing gum by managing various characteristics of the multiple ingredients, the delivery system containing the multiple ingredients, and/or the compressible chewing gum containing the delivery system and/or how the delivery system is made in a manner as previously discussed above.

The active ingredients mentioned above are meant as examples of active ingredients which could be applicable in a chewing gum, however, this list should not be considered as exhaustive.

Active ingredients to be applied in chewing gum according to embodiments of the invention may be applied as such or be included or bonded in different ways, such as being part of an inclusion complex e.g. as described in US 5,866,179, which is hereby incorporated by reference. A resin-bonding of nicotine is described in e.g. WO 2006/000232 which is also incorporated herein by reference. Further conventional methods of applying active ingredients may obviously be applied within the scope of the invention.

### EXAMPLE 1

### Preparation of gum base

A gum base is prepared, which comprises the following ingredients.

| Ingredients | Percent by weight |
|---|---|
| Elastomer | 10 |
| Natural resin | 28 |
| Synthetic resin | 22 |
| Fat/wax/emulsifiers | 23 |
| Fillers | 17 |

It should be emphasized that several other gum base compositions may be applied within the scope of the invention.

The elastomer and filler are added to a mixing kettle provided with mixing means like e.g. horizontally placed Z-shaped arms. The kettle has been preheated for 15 minutes to a temperature of about 120°C. The rubber is broken into small pieces and softened with mechanical action in the kettle.

The resin is slowly added to the elastomer until the mixture becomes homogeneous. The remaining resin is then added to the kettle and mixed for 10-20 minutes. The softening ingredients are added and mixed for 20-40 minutes until the whole mixture becomes homogeneous.

The mixture is then discharged into the pan and allowed to cool to room temperature from the discharged temperature of 120 °C.

### EXAMPLE 2

### Preparation of nicotine-containing chewing gum cores

Chewing gum cores are prepared by use of the gum base in example 1 and according to a conventional mechanical mixing procedure during moderate use of heating as described below.

| | |
|---|---|
| Gum base | 57.4% |
| Filler | 17.6% |
| Nicotine Polacrilex | |
| Nicotine | 0.2% |
| Ion exchange resin | 0.8% |
| Buffer agents | |
| Sodiumhydrogencarbonate | 1.0% |
| Sodium carbonate | 2.0% |
| | |
| Sorbitol powder | 19.1% |
| Liquid sweetener | 1.5% |
| Intense sweetener | 0.4% |

Gum base and filler are mixed in a mixing kettle provided with mixing means like e.g. horizontally placed Z-shaped arms. The kettle has been preheated to a temperature of up to approximately 50°C.

When the content is homogenous the other ingredients are added according to a specified time schedule. Nicotine is added in the first half of the mixing process and can be added as pure nicotine, as a nicotine salt or bound to an ion exchange resin, e.g. Amberlite IRP 64.

The chewing gum cores may be formulated with 0.1 - 8 mg of nicotine per piece preferably 2 or 4 mg. The pieces evaluated below comprise 2 mg nicotine complex.

### EXAMPLE 3

### Impregnation of nicotine-containing chewing gum cores with liquid flavor mixture

### Preparation stags:

Chewing gum cores of example 2 were placed in a coating pan and rotated for a few minutes.

### Impregnation stage:

Liquid flavor mixture, in this example essentially pure cinnamaldehyde (CA: cinnamaldehyde), was dosed into the coating pan through spray nozzles.

In this example, 6 doses were applied onto the chewing gum cores with a 3 minute pause in between each dose. Each dose contained about 0.2-0.25 %(w/w) of cinnamaldehyde pr. chewing gum core.

The concentration of cinnamaldehyde in the chewing gum core was measured by Gas Chromatography after extraction with solvent.

**Table 1: Analysis data. Chewing gum cores from example 3. CA concentration in %(w/w) of the chewing gum core.**

| | After dose 1 | After dose 2 | After dose 3 | After dose 4 | After dose 5 | After dose 6 |
|---|---|---|---|---|---|---|
| CA (%w/w) | 0.22 | 0.50 | 0.64 | 0.91 | 1.07 | 1.31 |

The cinnamaldehyde concentration impregnated onto the chewing gum core is retrieved in the measurements of the cinamaldehyde content of the chewing gum core.

### EXAMPLE 4

### Impregnation of nicotine-containing chewing gum cores with liquid flavor mixture

After preparation as described in example 2, impregnation was performed using a liquid flavor mixture comprising cinnamaldehyde (CA: cinnamaldehyde) and peppermint flavor.

In this case, 10 doses were applied onto the chewing gum cores with a 1 minute pause in between each dose. Each dose contained about 0.1 %(w/w) of cinnamaldehyde pr. chewing gum core and about 0.1 %(w/w) of peppermint pr. chewing gum core.

The same amount of flavor were added to the preparation in example 2 and compared to the impregnated cores. The taste of the core with flavor mixed into the core was comparable to the taste of the impregnated core.

### EXAMPLE 5

### Impregnation of nicotine-containing chewing gum cores with liquid flavor mixture

After preparation as described in example 2, impregnation was performed using a liquid flavor mixture containing lemon and another liquid flavor mixture containing cooling agent.

### Impregnation procedure

1 dose of lemon liquid flavor mixture was applied onto the chewing gum core and after 10 seconds, 1 dose of cooling agent liquid flavor mixture was added onto the chewing gum core.

Air was blown through the drum for 30 s, and the whole procedure was repeated 3 times.

Each dose of lemon liquid flavor mixture comprised about 0.5 %(w/w) of lemon pr. chewing gum core. Each dose of cooloing agent liquid flavor mixture contained about 0.1 %w/w of cooling agent pr. chewing gum core.

The same amount of flavor and cooling agent was added to the preparation in example 2 and compared to the impregnated cores. The taste of the core with the taste ingredients mixed into the core was comparable to the taste of the impregnated core.

### EXAMPLE 6

### Impregnation of nicotine-containing chewing gum cores with liquid flavor mixture

After preparation as described in example 2, impregnation was performed using a liquid flavor mixture containing wintergreen and another liquid flavor mixture containing dissolved menthol.

### Impregnation procedure

6 doses of wintergreen liquid flavor mixture were applied onto the chewing gum core with 30 s drying time in between each dose. After about 1 minute 1 dose of liquid flavor mixture containing menthol was added onto the chewing gum core.

Each dose of wintergreen liquid flavor mixture comprised about 0.1 %(w/w) of wintergreen pr. chewing gum core. The menthol liquid flavor mixture dose contained about 0.2 %w/w of menthol pr. chewing gum core.

The same amount of flavor was added to the preparation in example 2 and compared to the impregnated cores. The taste of the core with flavor mixed into the core was comparable to the taste of the impregnated core

### EXAMPLE 7

### Evaluation of examples 3-6

From table 1 it can be seen that the concentration of flavor in the chewing gum core correlates very well with the amount of flavor comprised in the liquid flavor mixture impregnated onto the chewing gum core. Consequently, the affinity of the flavor for the gum base in the chewing gum core and the resulting migration of flavor into the chewing gum core is shown.

Further examples have shown that similar patterns as measured for cinnamaldehyde are seen for a number of other flavors and optional components of the liquid flavor mixture, depending on the affinity for the chewing gum core of elements of the liquid flavor mixture.

It has been shown in the examples that the taste of chewing gum cores impregnated with flavors is comparable to the taste of chewing gum cores with the same flavor mixed into the cores.

### EXAMPLE 8

### Preparation of nicotine-containing chewing gum cores containing flavor

Chewing gum cores are prepared by use of the gum base in example 1 and according to a conventional mechanical mixing procedure during moderate use of heating as described below.

| | |
|---|---|
| Gum base: | 56.4% |
| Filler: | 16.6% |
| Nicotine Polacrilex: | |
| Nicotine | 0.2% |
| Ion exchange resin | 0.8% |
| Buffer agents: | |
| Sodiumhydrogencarbonate | 1.0% |
| Sodium carbonate | 2.0% |
| | |
| Sorbitol powder | 19.1% |
| Liquid sweetener | 1.5% |
| Intense sweetener | 0.4% |
| | |
| Flavor | 2.0% |

Gum base and filler are mixed in a mixing kettle provided with mixing means like e.g. horizontally placed Z-shaped arms. The kettle has been preheated to a temperature of up to approximately 50°C.

When the content is homogenous the other ingredients are added according to a specified time schedule. Nicotine is added in the first half of the mixing process and can be added as pure nicotine, as a nicotine salt or bound to an ion exchange resin, e.g. Amberlite IRP 64.

The chewing gum cores may be formulated with 0.1 - 8 mg of nicotine per piece preferably 2 or 4 mg. The pieces evaluated below comprise 2 mg nicotine complex.

### EXAMPLE 9

### Impregnation of flavor- and nicotine-containing chewing gum cores with liquid flavor mixture

Chewing gum cores of example 8 were placed in a coating pan and rotated for a few minutes. Small amounts of talc were added at this stage to prevent the chewing gum cores from sticking together.

Liquid flavor mixture, in this example essentially pure cinnamaldehyde, was dosed into the coating pan through spray nozzles. Air flow through the coating pan was used intermittently to remove volatiles.

In this example, 6 doses were applied onto the chewing gum cores with a 2 minute pause in between each dose. Each dose contained about 0.2 - 0.25 %(w/w) of cinnamaldehyde pr. chewing gum core.

The concentration of cinnamaldehyde in the chewing gum core was measured by Gas Chromatography after extraction with solvent.

A tendency similar to example 3 was seen: The flavor added during impregnation is to be found in the chewing gum core.

### EXAMPLE 10

### Impregnation of chewing gum cores using maltitol powder as a process aid in the impregnation step

The same impregnation procedure as in example 12 was used except that before (or in connection with) each dosage of liquid flavor mixture a small amount of maltitol powder was applied on the chewing gum cores to promote the immediate adsorption of the liquid flavor mixture and prevent the aggregation of chewing gum cores as a result of the stickiness of the liquid flavor mixture.

### EXAMPLE 11

### Coating of impregnated chewing gum cores

Impregnated chewing gum cores according to example 3-6, 9 and 10 were provided with a hard coating applied after the last impregnation dose.

The hard coatings were applied keeping the original shape of the chewing gum cores and the impregnation did not affect the hard coatings.

Optional coatings may e.g. be applied according to the methods disclosed in US patent 6,627,234, hereby incorporated by reference.

### EXAMPLE 12

### Impregnation of compressed chewing gum tablets with liquid flavor mixture

### Gum base from example 1 is processed into granules as follows:

To form the gum base as granules, the prepared gum base is transferred either directly or in the form of pellets to an extruder (here a Leistritz ZSE/BL 360 kw 104, available from Leistritz GmbH, Germany), which extrudes the gum base through a die plate into a liquid filled chamber (here a granulator A5 PAC 6, available from GALA GmbH, Germany). Descriptions of the extruder and the granulator may be found in e.g. WO 2004/098305, incorporated herein by reference.

The already prepared gum base composition is added at a first inlet of the extruder. The extruder delivers the gum base-comprising composition at a feed rate of 400 kg/h to the die plate. An extruder screw speed set at 247 rpm is applied, and the temperature in the extruder is in the range of 40°C to 70°C along about ¾ of the extruder barrel length, until the composition passes a heating device in the outlet end of the extruder. Here the composition is heated to an extruder exit temperature of about 109°C. The extruder and the granulator produce a pressure difference of about 70-75 bar.

The composition is extruded through the die plate, which is here a die plate having 696 holes with a diameter of 0.36 mm and being heated to a temperature of about 177°C. In the granulator chamber the extruded composition is cut to granules by a cutter with 8 blades and cutter speed set at 1999 rpm. The particles are cooled and transported to the strainer unit (here a centrifugal dryer TWS 20, available from GALA GmbH, Germany) in water with temperature about 11°C and flow about 22 m³/h. The average cooling and transport time in water is approx. 60 seconds. The particle rate is 400 kg/h and the average diameter of the obtained particles is here obtained to be 0.93 mm.

The cooling and transport stage carried out in water in this example could be carried out in other media such as e.g. air as well. Also various alternative apparatuses, die plates, settings, etc. could be applied in order to obtain smaller or larger average particle sizes of the prepared granules.

Gum base granules (40 % by weight of the total chewing gum tablet) were mixed with bulk sweetener (57.4%, by weight of the total chewing gum tablet), nicotine polacrilex (1.1% by weight of the total chewing gum tablet) and tabletting aid (1.5% by weight of the total chewing gum tablet).

The mixture was compressed into chewing gum tablets having a weight of approximately 1.5 g each.

The chewing gum tablets were impregnated with liquid flavor mixture in a process according to example 4, the compressed chewing gum tablets substituting the chewing gum cores.

The chewing gum tablet impregnated with liquid flavor mixture achieved a desired taste and preserved tablet integrity.

### EXAMPLE 13

### Impregnation of nicotine-containing chewing gum cores with liquid flavor mixture and coating the chewing gum cores with a coating comprising flavor

### Preparation starge.

Chewing gum cores of example 2 were placed in a coating pan and rotated for a few minutes.

### Impregnation stage:

Liquid flavor mixture, in this example essentially pure cinnamaldehyde (CA: cinnamaldehyde) with dissolved menthol, was dosed into the coating pan through spray nozzles. Air flow through the coating pan was used intermittently to remove volatiles.

In this example, 3 doses were applied onto the chewing gum cores with a 1 minute pause in between each dose. Each dose contained about 0.15 %(w/w) of liquid flavor mixture pr. chewing gum core.

Accordingly, a total of 0.45 %(w/w) of liquid flavor mixture was impregnated onto the chewing gum core.

The same amount of cinnamaldehyde comprised in a coating, that is 0.45 %(w/w) pr. chewing gum core, was applied on the chewing gum core.

After storage for 40 days under ambient conditions, the coating was removed from the core. The cinnamaldehyde content of the core was measured.

Measured content in core: 0.50 %(w/w)

From the measurements it can be seen that cinnamaldehyde impregnated onto the chewing gum core is to be found in the core.

Taste evaluation (comparing with the cinnalmalaldehyde mixed into the core) showed that the taste was comparable, confirming that flavor from the liquid flavor mixture migrated into the chewing gum core thereby increasing the amount of flavor in the chewing gum core to advantageous levels obtaining the desired taste profile.

### EXAMPLE 14

### Preparation of nicotine-containing chewing gum cores containing high amount of flavor

Chewing gum cores are prepared by use of the gum base in example 1 and according to a conventional mechanical mixing procedure.

| | |
|---|---|
| Gum base: | 56.4% |
| Filler: | 16.6% |
| Nicotine Polacrilex: | |
| Nicotine | 0.2% |
| Ion exchange resin | 0.8% |
| Buffer agents: | |
| Sodiumhydrogencarbonate | 1.0% |
| Sodium carbonate | 2.0% |
| Sorbitol powder | 17.1% |
| Liquid sweetener | 1.5% |
| Intense sweetener | 0.4% |
| Liquid flavor | 4.0% |

The mixture was treated as described in example 8 to obtain a homogeneous mixture. The mixture was rolled and scored but the product was so soft that it was not possible to obtain the correct weight and dimension of the chewing gum cores.

### EXAMPLE 15

### Preparation of nicotine-containing chewing gum cores containing high amount of flavor obtained by impregnation.

The amount of liquid flavor was reduced to 2% in the core compared to example 14:

| | |
|---|---|
| Gum base: | 56.4% |
| Filler: | 16.6% |
| Nicotine Polacrilex: | |
| Nicotine | 0.2% |
| Ion exchange resin | 0.8% |
| Buffer agents: | |
| Sodiumhydrogencarbonate | 1.0% |
| Sodium carbonate | 2.0% |
| Sorbitol powder | 19.1% |
| Liquid sweetener | 1.5% |
| Intense sweetener | 0.4% |
| Liquid flavor | 2.0% |

The mixture was mixed, rolled and scored as in example 14. The chewing gum cores prepared in this way met all specification concerning weight and dimension and contained about half the amount of flavor when compared to the cores of example 14.

The chewing gum cores were then placed in the coating pan. A liquid flavor mixture containing a total of about 2% by weight of the chewing gum cores of liquid flavor was applied to the chewing gum cores by impregnation in a process as described in example 4 and using maltitol as process aid to avoid stickiness which may damage the individual chewing gum cores.

A total about 4% liquid flavor in the chewing gum core was obtained using impregnation.

### EXAMPLE 16

### Evaluation of examples 14 and 15.

When comparing examples 14 and 15 it becomes evident that higher loadings of liquid flavor in the chewing gum core may be obtained by using the impregnation method herein described than would be possible by conventional mixing methods. In example 14, conventional mixing of liquid flavor with the other chewing gum core ingredients, before the chewing gum cores are formed, is used. The mixture contained about 4% of liquid flavor, but it was not possible to prepare suitable chewing gum cores from the mixture, because the product was far too soft and not easily processable.

In example 15, about half of the total amount of liquid flavor is mixed with the other ingredients in a mixer and suitable chewing gum cores were prepared from the mixture without difficulties. Then the other half of the liquid flavor is added to the prepared chewing gum cores by impregnation as defined herein. While the chewing gum cores prepared according to example 14 were not suitable for use as chewing gum or for further processing, the chewing gum cores prepared according to example 15 were ready for use or further processing. Approximately the same amount of flavor has been added to the chewing gum cores of example 14 and 15, respectively. For chewing gum where comparatively large amounts of flavor are needed in the chewing gum core, the impregnation process described herein thus has distinct advantages compared to a conventional mixing process.

### EXAMPLE 17

### Dimensional stability of chewing gum cores

The dimensions of 10 chewing gum cores from example 15 were carefully measured with a calliper 10 minutes after impregnation. The cores were numbered and placed on glass plates and measured again after 2 hours, 5 hours and 24 hours.

The dimensions of the chewing gum cores did not change significantly during 24 hours. The cores of example 14, holding approximately the same amount of flavor as the measured impregnated cores, could not even be measured due to the softness of the cores, confirming that higher loadings of flavor are possible with the impregnation method when compared to chewing gum cores with flavor in comparable amounts homogeneously mixed into the gum base.

## Claims

1. Method of adding flavor to a chewing gum core of a chewing gum
said chewing gum comprising active pharmaceutical ingredients,
said method of adding flavor to a chewing gum core comprising the steps of:
- providing a chewing gum core comprising gum base
- impregnating said chewing gum core by adding at least one dose of liquid flavor mixture onto the chewing gum core prior to any optional coating of the chewing gum core,
wherein at least the flavor in the liquid flavor mixture has affinity for the gum base in the chewing gum core and
wherein said chewing gum core comprises at least a part of said active pharmaceutical ingredient.

2. Method of adding flavor according to claim 1,
wherein said optional coating comprises at least a part of said active pharmaceutical ingredient.

3. Method of adding flavor according to any of the claims 1 to 2,
wherein said active pharmaceutical ingredient is nicotine.

4. Method of adding flavor according to any of the claims 1-3, wherein said impregnation with said liquid flavor mixture is performed using multiple doses.

5. Method of adding flavor according to any of the claims 1 to 4,
wherein each of said doses comprises 0.05 to 2.0 mg, 2.0 to 5.0 mg, or 5.0 to 50 mg of liquid flavor mixture pr. chewing gum core.

6. Method of adding flavor according to any of the claims 1 to 5,
wherein said liquid flavor mixture comprises flavor in an amount of above 20, preferably above 40, most preferably above 60% by weight of the liquid flavor mixture.

7. Method of adding flavor according to any of the claims 1 to 6, wherein said optional coating is selected from the group consisting of a hard coating, a soft coating, a film coating, a gel coating or any combination thereof.

8. Method of adding flavor according to any of the claims 1 to 7, wherein said liquid flavor mixture comprises at least two different flavors.

9. Method of adding flavor according to any of the claims 1 to 8,
wherein said liquid flavor mixture comprises flavor selected from the group consisting of cinnamon, wintergreen, spearmint, orange and lemon.

10. Method of adding flavor according to any of the claims 1 to 9, wherein said liquid flavor mixture comprises softener, emulsifier, high intensity sweetener, sweetener, anti-oxidants or any combinations thereof.

11. Method of adding flavor according to any of the claims 1 to 10, wherein said chewing gum core comprises flavor prior to impregnation.

12. Method of adding flavor according to any of the claims 1 to 11,
wherein said chewing gum core is compressed.

13. Method of adding flavor according to any of the claims 1 to 12,
wherein said chewing gum core is at least partly covered with powdery process aids prior to and/or during impregnation, said powdery process aid is selected from the group consisting of polyol sugars such as maltitol, calcium carbonate, talc, high intensity sweeteners, solid flavor and combinations thereof.

14. Method of adding flavor according to any of the claims 1 to 13 whereby the release of flavor from a chewing gum is adjusted by
storing said chewing gum under controlled conditions for at least 1 week, said controlled conditions comprise a temperature between 5-30°C, preferably between 10-25°C.

15. A chewing gum comprising active pharmaceutical ingredients and flavor, wherein there is an inhomogeneous distribution of flavor in the chewing gum core and wherein the concentration of flavor is highest in the peripheral areas of the chewing gum core and wherein the inhomogeneous distribution of flavor provides improved dimensional stability to the chewing gum core, said chewing gum being obtainable by a process wherein the flavor is added by the method of any of the claims 1 - 14.

## Patentansprüche

1. Verfahren zum Hinzufügen von Geschmack oder Aroma zu einem Kaugummikern eines Kaugummis,
wobei der Kaugummi aktive pharmazeutische Inhaltsstoffe umfasst,
wobei das Verfahren zum Hinzufügen von Geschmack oder Aroma zu einem Kaugummikern die Schritte umfasst:
- Bereitstellen eines Kaugummikerns, welcher Gummigrundlage umfasst,
- Imprägnieren des Kaugummikerns durch Hinzugeben von mindestens einer Dosis von flüssiger Geschmack- oder Aromastoffmischung auf den Kaugummikern vor einem jeglichen optionalen Überziehen des Kaugummikerns,
wobei mindestens der Geschmack- oder Aromastoff in der flüssigen Geschmack- oder Aromastoffmischung eine Affinität für die Gummigrundlage in dem Kaugummikern aufweist und
wobei der Kaugummikern mindestens einen Teil des aktiven pharmazeutischen Inhaltsstoffs umfasst.

2. Verfahren zum Hinzufügen von Geschmack oder Aroma nach Anspruch 1, wobei der optionale Überzug mindestens einen Teil des aktiven pharmazeutischen Inhaltsstoffs umfasst.

3. Verfahren zum Hinzufügen von Geschmack oder Aroma nach einem der Ansprüche 1 bis 2, wobei der aktive pharmazeutische Inhaltsstoff Nicotin ist.

4. Verfahren zum Hinzufügen von Geschmack oder Aroma nach einem der Ansprüche 1 bis 3, wobei die Imprägnierung mit der flüssigen Geschmack- oder Aromastoffmischung unter Verwendung einer Mehrzahl von Dosen ausgeführt wird.

5. Verfahren zum Hinzufügen von Geschmack oder Aroma nach einem der Ansprüche 1 bis 4, wobei jede der Dosen 0,05 bis 2,0 mg, 2,0 bis 5,0 mg oder 5,0 bis 50 mg flüssige Geschmack- oder Aromastoffmischung pro Kaugummikern umfasst.

6. Verfahren zum Hinzufügen von Geschmack oder Aroma nach einem der Ansprüche 1 bis 5, wobei die flüssige Geschmack- oder Aromastoffmischung Geschmack oder Aroma in einer Menge von über 20, vorzugsweise über 40, am meisten bevorzugt über 60 Gew.-% der flüssigen Geschmack- oder Aromastoffmischung umfasst.

7. Verfahren zum Hinzufügen von Geschmack oder Aroma nach einem der Ansprüche 1 bis 6, wobei der optionale Überzug aus der Gruppe bestehend aus einem harten Überzug, einem weichen Überzug, einem Filmüberzug, einem Gelüberzug oder einer jeglichen Kombination davon ausgewählt ist.

8. Verfahren zum Hinzufügen von Geschmack oder Aroma nach einem der Ansprüche 1 bis 7, wobei die flüssige Geschmack- oder Aromastoffmischung mindestens zwei unterschiedliche Geschmack- bzw. Aromastoffe umfasst.

9. Verfahren zum Hinzufügen von Geschmack oder Aroma nach einem der Ansprüche 1 bis 8, wobei die flüssige Geschmack- oder Aromastoffmischung Geschmack oder Aroma, ausgewählt aus der Gruppe bestehend aus Zimt, Wintergrünöl, Krauseminze, Orange und Zitrone, umfasst.

10. Verfahren zum Hinzufügen von Geschmack oder Aroma nach einem der Ansprüche 1 bis 9, wobei die flüssige Geschmack- oder Aromastoffmischung Weichmacher, Emulgiermittel, hochintensiven Süßstoff, Süßstoff, Antioxidationsmittel oder jegliche Kombinationen davon umfasst.

11. Verfahren zum Hinzufügen von Geschmack oder Aroma nach einem der Ansprüche 1 bis 10, wobei der Kaugummikern Geschmack oder Aroma vor der Imprägnierung umfasst.

12. Verfahren zum Hinzufügen von Geschmack oder Aroma nach einem der Ansprüche 1 bis 11, wobei der Kaugummikern verdichtet ist bzw. wird.

13. Verfahren zum Hinzufügen von Geschmack oder Aroma nach einem der Ansprüche 1 bis 12, wobei der Kaugummikern mindestens teilweise mit pulverförmigen Verfahrenshilfsstoffen vor und/oder während der Imprägnierung bedeckt wird, wobei der pulverförmige Verfahrenshilfsstoff aus der Gruppe bestehend aus Polyolzuckern, wie Maltitol, Calciumcarbonat, Talk, hochintensiven Süßstoffen, festen Geschmack- oder Aromastoffen und Kombinationen davon ausgewählt ist.

14. Verfahren zum Hinzufügen von Geschmack oder Aroma nach einem der Ansprüche 1 bis 13, wobei die Freisetzung von Geschmack- oder Aromastoff aus einem Kaugummi angepasst wird durch Aufbewahren des Kaugummis unter kontrollierten Bedingungen für mindestens 1 Woche, wobei die kontrollierten Bedingungen eine Temperatur zwischen 5 und 30°C, vorzugsweise zwischen 10 und 25°C umfassen.

15. Kaugummi, welcher aktive pharmazeutische Inhaltsstoffe und Geschmack oder Aroma umfasst, worin es eine inhomogene Verteilung von Geschmack oder Aroma in dem Kaugummikern gibt und wobei die Konzentration von Geschmack oder Aroma in den peripheren Flächen des Kaugummikerns am höchsten ist und wobei die inhomogene Verteilung von Geschmack oder Aroma dem Kaugummikern eine verbesserte Formstabilität verleiht, wobei der Kaugummi erhalten werden kann durch ein Verfahren, in welchem der Geschmack oder das Aroma durch das Verfahren nach einem der Ansprüche 1-14 hinzugefügt wird.

## Revendications

1. Procédé d'ajout d'arôme à un noyau de gomme à mâcher d'une gomme à mâcher, ladite gomme à mâcher comprenant des principes actifs pharmaceutiques, ledit procédé d'ajout d'arôme à un noyau de gomme à mâcher comprenant les étapes suivantes :
- la fourniture d'un noyau de gomme à mâcher comprenant une base de gomme,
- l'imprégnation dudit noyau de gomme à mâcher par l'ajout d'au moins une dose de mélange d'arômes liquide dans le noyau de gomme à mâcher avant un quelconque enrobage éventuel du noyau de gomme à mâcher,
dans lequel au moins l'arôme dans le mélange d'arômes liquide présente une affinité pour la base de gomme dans le noyau de gomme à mâcher et
dans lequel ledit noyau de gomme à mâcher comprend au moins une partie dudit principe actif pharmaceutique.

2. Procédé d'ajout d'arôme selon la revendication 1,
dans lequel ledit enrobage optionnel comprend au moins une partie dudit principe actif pharmaceutique.

3. Procédé d'ajout d'arôme selon l'une quelconque des revendications 1 à 2,
dans lequel ledit principe actif pharmaceutique est de la nicotine.

4. Procédé d'ajout d'arôme selon l'une quelconque des revendications 1 à 3,
dans lequel ladite imprégnation avec ledit mélange d'arômes liquide est réalisée au moyen de multiples doses.

5. Procédé d'ajout d'arôme selon l'une quelconque des revendications 1 à 4,
dans lequel chacune desdites doses comprend de 0,05 à 2,0 mg, de 2,0 à 5,0 mg ou de 5,0 à 50 mg de mélange d'arômes liquide par noyau de gomme à mâcher.

6. Procédé d'ajout d'arôme selon l'une quelconque des revendications 1 à 5,
dans lequel ledit mélange d'arômes liquide comprend de l'arôme dans une quantité supérieure à 20, de préférence supérieure à 40, et de façon davantage préférée supérieure à 60 % en poids du mélange d'arômes liquide.

7. Procédé d'ajout d'arôme selon l'une quelconque des revendications 1 à 6, dans lequel ledit enrobage optionnel est sélectionné parmi le groupe composé d'un enrobage dur, d'un enrobage souple, d'un enrobage par film, d'un enrobage sous forme de gel ou d'une quelconque combinaison de ceux-ci.

8. Procédé d'ajout d'arôme selon l'une quelconque des revendications 1 à 7, dans lequel ledit mélange d'arômes liquide comprend au moins deux arômes différents.

9. Procédé d'ajout d'arôme selon l'une quelconque des revendications 1 à 8,
dans lequel ledit mélange d'arômes liquide comprend un arôme sélectionné parmi le groupe composé de cannelle, de gaulthérie, de menthe verte, d'orange et de citron.

10. Procédé d'ajout d'arôme selon l'une quelconque des revendications 1 à 9, dans lequel ledit mélange d'arômes liquide comprend un ramollissant, un émulsifiant, un édulcorant à haute intensité, un édulcorant, des antioxydants ou quelconques combinaisons de ceux-ci.

11. Procédé d'ajout d'arôme selon l'une quelconque des revendications 1 à 10, dans lequel ledit noyau de gomme à mâcher comprend un arôme avant l'imprégnation.

12. Procédé d'ajout d'arôme selon l'une quelconque des revendications 1 à 11,
dans lequel ledit noyau de gomme à mâcher est comprimé.

13. Procédé d'ajout d'arôme selon l'une quelconque des revendications 1 à 12,
dans lequel ledit noyau de gomme à mâcher est au moins partiellement recouvert d'auxiliaires de traitement sous forme de poudre avant et/ou pendant l'imprégnation, ledit auxiliaire de traitement sous forme de poudre étant sélectionné parmi le groupe composé de sucres de polyol tels que du maltitol, du carbonate de calcium, du talc, des édulcorants à haute intensité, des arômes solides et des combinaisons de ceux-ci.

14. Procédé d'ajout d'arôme selon l'une quelconque des revendications 1 à 13, selon lequel la libération d'arôme depuis une gomme à mâcher est réglée par le stockage de ladite gomme à mâcher dans des conditions régulées pendant au moins 1 semaine, lesdites conditions régulées comprenant une température entre 5 et 30 °C, de préférence entre 10 et 25 °C.

15. Gomme à mâcher comprenant des principes actifs pharmaceutiques et un arôme, dans laquelle il existe une répartition hétérogène d'arôme dans le noyau de gomme à mâcher et dans laquelle la concentration d'arôme est la plus élevée dans les régions périphériques du noyau de gomme à mâcher et dans laquelle la répartition hétérogène d'arôme fournit une stabilité dimensionnelle améliorée au noyau de gomme à mâcher, ladite gomme à mâcher pouvant être obtenue par un procédé dans lequel l'arôme est ajouté au moyen du procédé selon l'une quelconque des revendications 1 à 14.
